# EUROPÄISCHE PATENTSCHRIFT

(11) **EP 0 576 970 B1**
(45) Veröffentlichungstag und Bekanntmachung des Hinweises auf die Patenterteilung: **14.01.1998**
(21) Anmeldenummer: 93109966.7
(22) Anmeldetag: 22.06.1993
(51) Int. Cl.: C07F 17/00, C08F 10/00

(54) **Metallocene mit arylsubstituierten Indenylderivaten als Liganden, Verfahren zu ihrer Herstellung und ihre Verwendung als Katalysatoren**
Metallocenes with arylsubstituted indenyl-derivatives as ligands, process for their preparation and their use as catalysts
Métallocènes contenant des dérivés d'indène aryl-substitué comme ligands, procédé de préparation et application comme catalyseurs

(30) Priorität: 27.06.1992 DE 4221244
(43) Veröffentlichungstag der Anmeldung: 05.01.1994
(60) Teilanmeldung: 97107297.0
(73) Patentinhaber: HOECHST AKTIENGESELLSCHAFT, 65926 Frankfurt am Main (DE)
(72) Erfinder: Küber, Frank, Dr., D-6370 Oberursel (DE); Bachmann, Bernd, Dr., D-6239 Eppstein/Ts. (DE); Spaleck, Walter, Dr., D-6237 Liederbach (DE); Winter, Andreas, Dr., D-6246 Glashütten/Ts. (DE); Rohrmann, Jürgen, Dr., D-6233 Kelkheim/Ts. (DE)

(56) Entgegenhaltungen:
- EP-A- 0 344 887
- EP-A- 0 485 821
- EP-A- 0 485 823
- EP-A- 0 530 647

## Beschreibung

Die vorliegende Erfindung bezieht sich auf neue Metallocene mit arylsubstituierten Indenylderivaten als Liganden, die sehr vorteilhaft als Katalysatorkomponenten bei der Herstellung von Polyolefinen mit hoher Isotaktizität, enger Molmassenverteilung und sehr hoher Molmasse verwendet werden können.

Polyolefine mit hoher Molmasse besitzen insbesondere Bedeutung für die Herstellung von Folien, Platten oder Großhohlkörpern oder Formteilen, wie beispielsweise Rohren.

Aus der Literatur ist die Herstellung von Polyolefinen mit löslichen Metallocenverbindungen in Kombination mit Aluminoxanen oder anderen Cokatalysatoren, die aufgrund ihrer Lewis-Acidität das neutrale Metallocen in ein Kation überführen und stabilisieren können, bekannt.

Lösliche Metallocenverbindungen auf der Basis von Bis(cyclopentadienyl)zirkon-dialkyl bzw. dihalogenid in Kombination mit oligomeren Aluminoxanen können Ethylen mit guter und Propylen mit mäßiger Aktivität polymerisieren. Man erhält Polyethylen mit enger Molmassenverteilung und mittlerer Molmasse. Das auf diese Weise hergestellte Polypropylen ist ataktisch und hat eine sehr niedrige Molmasse.

Die Herstellung von isotaktischem Polypropylen gelingt mit Hilfe des Ethylenbis(4,5,6,7-tetrahydro-1-indenyl)zirkoniumdichlorids zusammen mit einem Aluminoxan in einer Suspensionspolymerisation (vgl. EP 185 918). Das Polymer besitzt eine enge Molmassenverteilung. Besonderer Nachteil dieses Verfahrens ist, daß bei technisch relevanten Polymerisationstemperaturen nur Polymere mit sehr niedriger Molmasse hergestellt werden können.

Es wurde auch eine spezielle Voraktivierungsmethode des Metallocens mit einem Aluminoxan vorgeschlagen, welche zu einer beachtlichen Steigerung der Aktivität des Katalysatorsystems und zu einer deutlichen Verbesserung der Kornmorphologie des Polymeren führt (vgl. DE 37 26 067). Die Voraktivierung erhöht die Molmasse jedoch nicht wesentlich.

Weiterhin sind Katalysatoren auf der Basis von Ethylenbisindenylhafniumdichlorid und Ethylenbis(4,5,6,7-tetrahydro-1-indenyl)hafniumdichlorid und Methylaluminoxan bekannt, mit denen durch Suspensionspolymerisation höhermolekulare Polypropylene hergestellt werden können (vgl. J. Am. Chem. Soc. (1987), 109, 6544). Unter technisch relevanten Polymerisationsbedingungen ist jedoch die Kornmorphologie der derart erzeugten Polymere nicht befriedigend und die Aktivität der eingesetzten Katalysatorsysteme vergleichsweise gering. Verbunden mit den hohen Katalysatorkosten ist somit mit diesen Systemen eine kostengünstige Polymerisation nicht möglich.

Eine deutliche Steigerung der Molmasse konnte durch die Verwendung von Metallocenen erreicht werden, bei denen die durch eine Brücke fixierten aromatischen π-Liganden in 2-Stellung (vgl. DE 40 35 886) oder in 2- und 4-Stellung (vgl. DE 41 28 238) Substituenten tragen.

Eine weitere Steigerung der Molmasse wurde durch die Verwendung aromatischer π-Liganden mit Substituenten in 2-, 4- und 6-Stellung (vgl. DE 41 39 596) sowie aromatischer *π*-Liganden vom 4,5- Benzoindenyltyp erreicht (vgl. DE 41 39 595).

Die letztgenannten Metallocene mit den genannten Substituenten sind in dieser Hinsicht bei einer Polymerisationstemperatur von 70°C bereits sehr leistungsfähig. Trotzdem sind die erzielbaren Molmassen bei der technisch optimalen Polymerisationstemperatur von 70°C für viele technische Anwendungen wie beispielsweise die Herstellung von Polymeren für Rohre und Großhohlkörper sowie spezielle Fasern noch zu gering.

Unter dem Zwang großtechnisch kostengünstiger Produktion muß bei möglichst hohen Reaktionstemperaturen polymerisiert werden, da bei höheren Polymerisationstemperaturen die entstehende Reaktionswärme mit weniger Kühlmedium abgeführt werden kann. Daher kann der Kühlwasserkreislauf deutlich geringer dimensioniert werden.

Ein häufig auftretender Nachteil der löslichen (homogenen) Metallocen-/Methylaluminoxan-Katalysatorsysteme in Verfahren, bei denen das gebildete Polymer als Feststoff anfällt, ist die Ausbildung von starken Belägen an Reaktorwänden und Rührer. Diese Beläge entstehen durch Agglomeration der Polymerpartikel, wenn das Metallocen, oder Aluminoxan, oder beide gelöst im Suspensionsmedium vorliegen. Derartige Beläge in den Reaktorsystemen müssen regelmäßig entfernt werden, da diese rasch erhebliche Stärken erreichen, eine hohe Festigkeit besitzen und den Wärmeaustausch zum Kühlmedium verhindern.

Es ist daher vorteilhaft, Metallocene in geträgerter Form einzusetzen. Ein effizientes und einfaches Verfahren zur Trägerung von Metallocenen, das universell in allen Polymerisationsverfahren einsetzbar ist, ist vorgeschlagen worden (vgl.EP-A-578 838).

Ein weiterer Nachteil im Fall der stereospezifischen Polymerisation prochiraler Monomere, z.B. von Propylen, mit Metallocenkatalysatoren ist die relativ niedrige Isotaxie, die sich im Falle von isotaktischem Polypropylen in niedrigen Schmelzpunkten auswirkt. Insbesondere Metallocene mit Substituenten in 2- und 4-Stellung und speziell rac-Dimethylsilylbis(2-Methyl-4-isopropyl-indenyl)zirkondichlord in Kombination mit Methylaluminoxan liefern im Fall von Propylen ein Polymer mit hoher Isotaktizität und daher hohem Schmelzpunkt (vgl. EP-A-530 647). Trotzdem sind die erzielbaren Schmelzpunkte bei technisch relevanten Polymerisationstemperaturen (z.B. 70°C) für einige technische Anwendungen zu niedrig.

Es gibt allerdings auch technische Anwendungen, bei denen niedrige Schmelzpunkte erwünscht sind.

Es bestand die Aufgabe, ein Verfahren und/oder ein Katalysatorsystem zu finden, das Polymere mit sehr hoher Molmasse und im Fall der isospezifischen Polymerisation prochiraler Monomere Polymere mit hoher Isotaxie in großer Ausbeute erzeugt. Durch Trägerung könnten die aus dem Stand der Technik bekannten Nachteile durch Belagsbildung und hohen Feinkornanteil vermieden werden. Durch Verwendung von Wasserstoff als Molmassenregler sollte dann der gesamte Bereich der technisch interessanten Molmassen mit nur einem Metallocen abgedeckt werden können.

Es wurde nun gefunden, daß Metallocene mit speziellen Indenylderivaten als Liganden geeignete Katalysatoren (Katalysatorkomponenten) bei der Herstellung von Polyolefinen mit hoher Molmasse, insbesondere bei Verwendung prochiraler Monomere von isotaktischen Polyolefinen mit sehr hoher Molmasse und sehr hoher Isotaxie sind.

Durch Umsetzung dieser löslichen Metallocene mit einer geträgerten aluminiumorganischen Katalysator-Komponente entsteht ein Katalysatorsystem, das zur Aktivierung keinen zusätzlichen Cokatalysator benötigt und die Ausbildung von Reaktorbelägen vollständig vermeidet.

Die vorliegende Erfindung betrifft daher Verbindungen der Formel I: 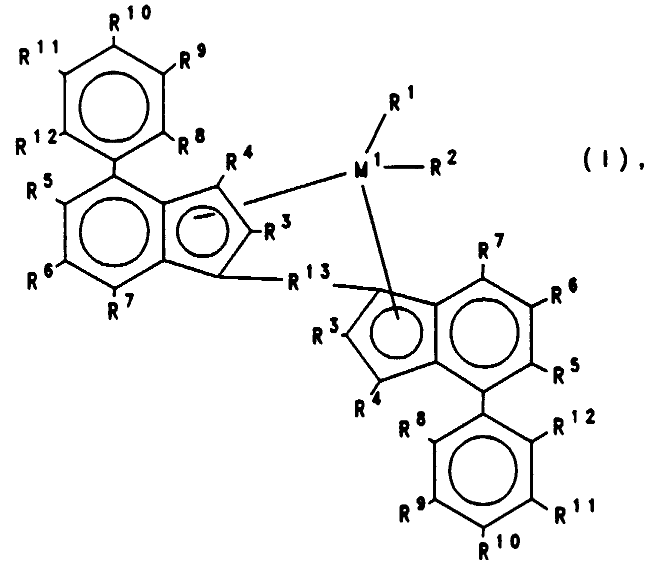 worin
M¹ ein Metall der Gruppe IVb, Vb oder Vlb des Periodensystems ist,
R¹ und R² gleich oder verschieden sind und ein Wasserstoffatom, eine C₁-C₁₀-Alkyl-, eine C₁-C₁₀-Alkoxy-, eine C₆-C₁₀-Aryl-, eine C₆-C₁₀-Aryloxy-, eine C₂-C₁₀-Alkenyl-, eine C₇-C₄₀-Arylalkyl-, eine C₇-C₄₀-Alkylaryl-, eine C₈-C₄₀-Arylalkenyl-, eine OH-Gruppe oder ein Halogenatom bedeuten,
die Reste R³ gleich oder verschieden sind und ein Wasserstoffatom, ein Halogenatom, eine C₁-C₁₀-Alkylgruppe, die halogeniert sein kann, eine C₆-C₁₀-Arylgruppe, einen NR¹⁶₂-, -SR¹⁶-, -OSiR¹⁶₃-, -SiR¹⁶₃- oder -PR¹⁶₂-Rest bedeuten, worin R¹⁶ ein Halogenatom, eine C₁-C₁₀-Alkylgruppe oder eine C₆-C₁₀-Arylgruppe ist,
R⁴ bis R¹² gleich oder verschieden sind und die für R³ genannten Bedeutungen besitzen, oder benachbarte Reste R⁴ bis R¹² mit den sie verbindenden Atomen einen oder mehrere aromatische oder aliphatische Ringe bilden, oder die Reste R⁵ und R⁸ oder R¹² mit den sie verbindenden Atomen einen aromatischen oder aliphatischen Ring bilden,
R¹³ 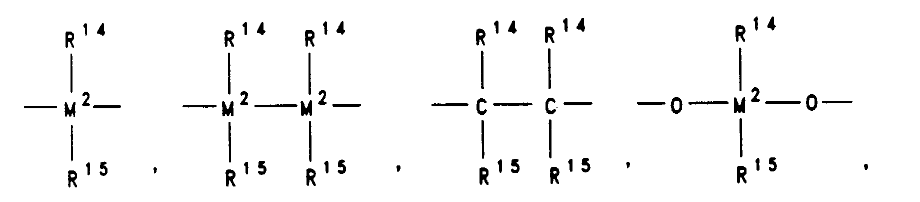 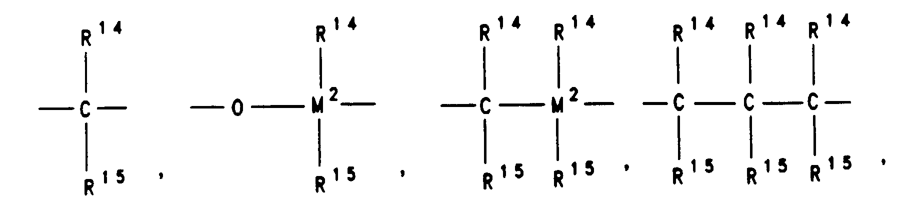 =BR¹⁴, =AlR¹⁴ -Ge-, -O-, -S-, =SO, =SO₂, =NR¹⁴, =CO, =PR¹⁴ oder =P(O)R¹⁴ ist, wobei R¹⁴ und R¹⁵ gleich oder verschieden sind und ein Wasserstoffatom, ein Halogenatom, eine C₁-C₁₀-Alkyl-, eine C₁-C₁₀-Fluoralkyl-, eine C₁-C₁₀-Alkoxy-, eine C₆-C₁₀-Aryl-, eine C₆-C₁₀-Fluoraryl-, eine C₆-C₁₀-Aryloxy-, eine C₂-C₁₀-Alkenyl-, eine C₇-C₄₀-Arylalkyl-, eine C₇-C₄₀-Alkylaryl-, eine C₈-C₄₀-Arylalkenylgruppe bedeuten, oder R¹⁴ und R¹⁵ jeweils mit den sie verbindenden Atomen einen oder mehrere Ringe bilden und
M² Silizium, Germanium oder Zinn ist.

Die vorliegende Erfindung betrifft auch ein Verfahren zur Herstellung eines Olefinpolymers durch Polymerisation oder Copolymerisation eines Olefins der Formel R^{a}-CH=CH-R^{b}, worin R^{a} und R^{b} gleich oder verschieden sind und ein Wasserstoffatom oder einen Kohlenwasserstoffrest mit 1 bis 14 C-Atomen bedeuten, oder R^{a} und R^{b} mit den sie verbindenden Atomen einen oder mehrere Ringe bilden können, bei einer Temperatur von -60 bis 200°C, bei einem Druck von 0,5 bis 100 bar, in Lösung, in Suspension oder in der Gasphase, in Gegenwart eines Katalysators, welcher aus einem Metallocen als Übergangsmetallverbindung und einem Cokatalysator gebildet wird, dadurch gekennzeichnet, daß das Metallocen eine Verbindung der Formel I ist.

Die erfindungsgemäßen Verbindungen sind Metallocene der Formel I 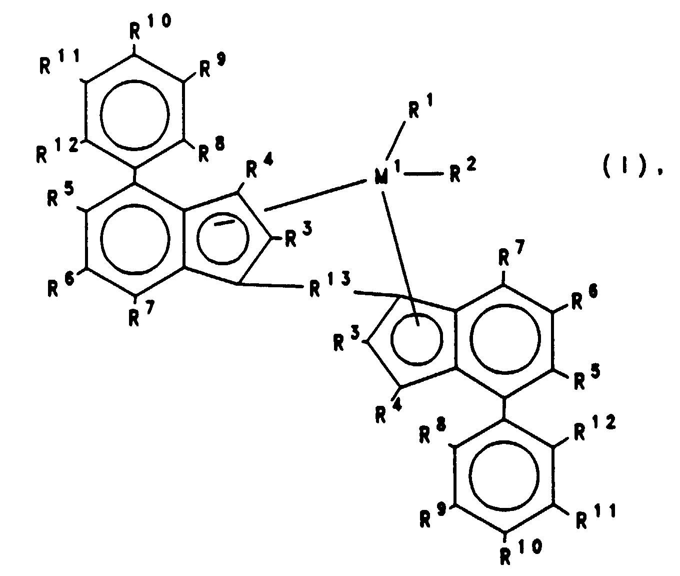 worin M¹ ein Metall der Gruppe IVb, Vb oder VIb des Periodensystems, beispielsweise Titan, Zirkonium, Hafnium, Vanadium, Niob, Tantal, Chrom, Molybdän oder Wolfram, vorzugsweise Zirkonium, Hafnium und Titan ist.

R¹ und R² sind gleich oder verschieden und bedeuten ein Wasserstoffatom, eine C₁-C₁₀-, vorzugsweise C₁-C₃-Alkylgruppe, eine C₁-C₁₀-,vorzugsweise C₁-C₃-Alkoxygruppe, eine C₆-C₁₀-, vorzugsweise C₆-C₈-Arylgruppe, eine C₆-C₁₀-, vorzugsweise C₆-C₈-, Aryloxygruppe, eine C₂-C₁₀-, vorzugsweise C₂-C₄-Alkenylgruppe, eine C₇-C₄₀-, vorzugsweise C₇-C₁₀-Arylalkylgruppe, eine C₇-C₄₀-, vorzugsweise C₇-C₁₂-Alkylarylgruppe, eine C₈-C₄₀-, vorzugsweise C₈-C₁₂-Arylalkenylgruppe, oder ein Halogenatom, vorzugsweise Chlor.

Die Reste R³ bis R¹² sind gleich oder verschieden und bedeuten ein Wasserstoffatom, ein Halogenatom, bevorzugt Fluor, Chlor oder Brom, eine C₁-C₁₀-, vorzugsweise C₁-C₄-Alkylgruppe, die halogeniert sein kann, eine C₆-C₁₀-, vorzugsweise C₆-C₈-Arylgruppe, einen - NR¹⁶₂-, -SR¹⁶-, -OSiR¹⁶₃-, -SiR¹⁶₃- oder -PR¹⁶₂-Rest, wobei R¹⁶ ein Halogenatom, vorzugsweise Chlor, oder eine C₁-C₁₀-, vorzugsweise C₁-C₄-Alkylgruppe oder eine C₆-C₁₀-, vorzugsweise C₆-C₈-Arylgruppe sein kann.

Die benachbarten Reste R⁴ bis R¹² können mit den sie verbindenden Atomen einen aromatischen, vorzugsweise 6-gliedrigen aromatischen oder aliphatischen, vorzugsweise 4-8-gliedrigen aliphatischen Ring bilden.
R¹³ ist 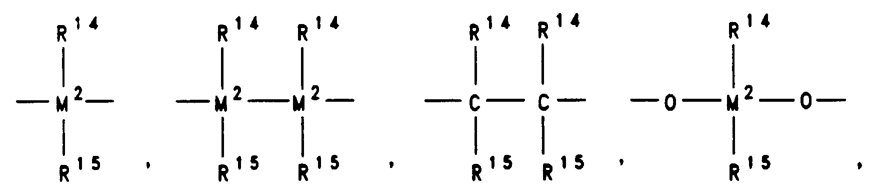 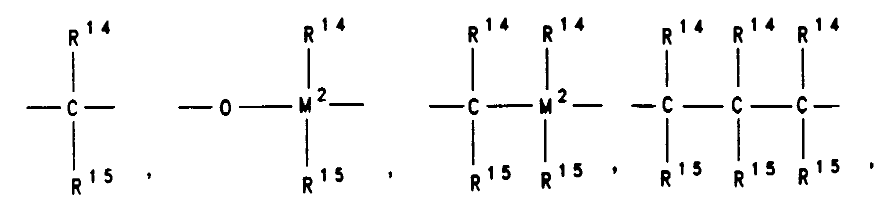 =BR¹⁴, =AIR¹⁴, -Ge-, -O-, -S-, =SO, =SO₂, =NR¹⁴, =CO, =PR¹⁴ oder =P(O)R¹⁴, vorzugsweise 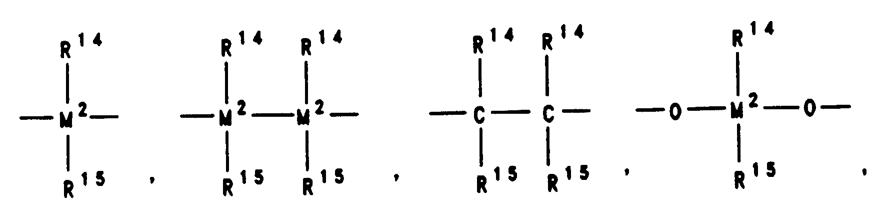 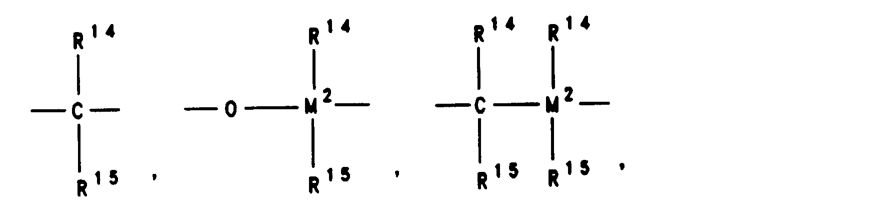 =BR¹⁴, =AlR¹⁴, -Ge-, -O-, -S-, =SO, =SO₂, =NR¹⁴, =CO, =PR¹⁴, oder =P(O)R¹⁴, wobei R¹⁴ und R¹⁵ gleich oder verschieden sind und ein Wasserstoffatom, ein Halogenatom, eine C₁-C₁₀-, vorzugsweise C₁-C₄-Alkylgruppe insbesondere Methylgruppe, eine C₁-C₁₀-Fluoralkyl-, vorzugsweise CF₃-Gruppe, eine C₆-C₁₀-, vorzugsweise C₆-C₈-Aryl-, eine C₆- C₁₀-Fluoraryl-, vorzugsweise Pentafluorphenylgruppe, eine C₁-C₁₀-, vorzugsweise C₁-C₄-Alkoxygruppe, insbesondere Methoxygruppe, eine C₂-C₁₀-, vorzugsweise C₂-C₄-Alkenylgruppe, eine C₇-C₄₀-, vorzugsweise C₇-C₁₀-Arylalkylgruppe, eine C₈-C₄₀-, vorzugsweise C₈-C₁₂-Arylalkenylgruppe, eine C₇-C₄₀-, vorzugsweise C₇-C₁₂-Alkylarylgruppe bedeuten oder R¹⁴ und R¹⁵ jeweils mit den sie verbindenden Atomen einen Ring bilden.
M² ist Silizium, Germanium oder Zinn, vorzugsweise Silizium oder Germanium.
Für Verbindungen der Formel I gilt bevorzugt, daß
M¹ Zirkonium oder Hafnium ist,
R¹ und R² gleich sind und eine C₁-C₃-Alkylgruppe oder ein Halogenatom bedeuten, die Reste R³ gleich sind und eine C₁-C₄-Alkylgruppe bedeuten,
R⁴ bis R¹² gleich oder verschieden sind und Wasserstoff oder eine C₁-C₄-Alkylgruppe bedeuten,
R¹³ für 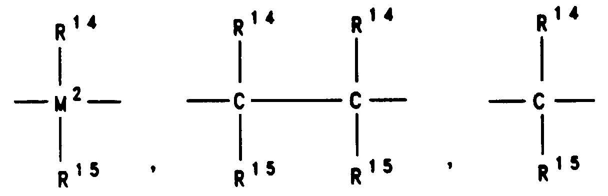 steht, wobei M² Silizium oder Germanium ist und R¹⁴ und R¹⁵ gleich oder verschieden sind und für eine C₁-C₄- Alkylgruppe oder eine C₆-C₁₀-Arylgruppe stehen.

Weiterhin bevorzugt sind Verbindungen der Formel I, bei denen die Reste R⁴ und R⁷ Wasserstoff bedeuten und R⁵, R⁶ und R⁸ bis R¹² für eine C₁-C₄-Alkylgruppe oder Wasserstoff stehen.

Besonders bevorzugt sind Verbindungen der Formel I, bei denen M¹ Zirkon ist, R¹ und R² gleich sind und Chlor bedeuten, die Reste R³ gleich sind und eine C₁-C₄-Alkylgruppe bedeuten, R⁴ und R⁷ für Wasserstoff steht, R⁵, R⁶ und R⁸ bis R¹² gleich oder verschieden und eine C₁-C₄- Alkylgruppe oder Wasserstoff bedeuten und R¹³ für 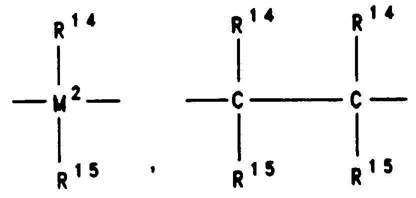 steht, wobei M² Silizium bedeutet, und R¹⁴ und R¹⁵ gleich oder verschieden sind und für eine C₁-C₄-Alkylgruppe oder eine C₆-C₁₀-Arylgruppe stehen.

Die Herstellung der Metallocene I erfolgt nach literaturbekannten Verfahren und ist im nachfolgenden Reaktionsschema wiedergegeben 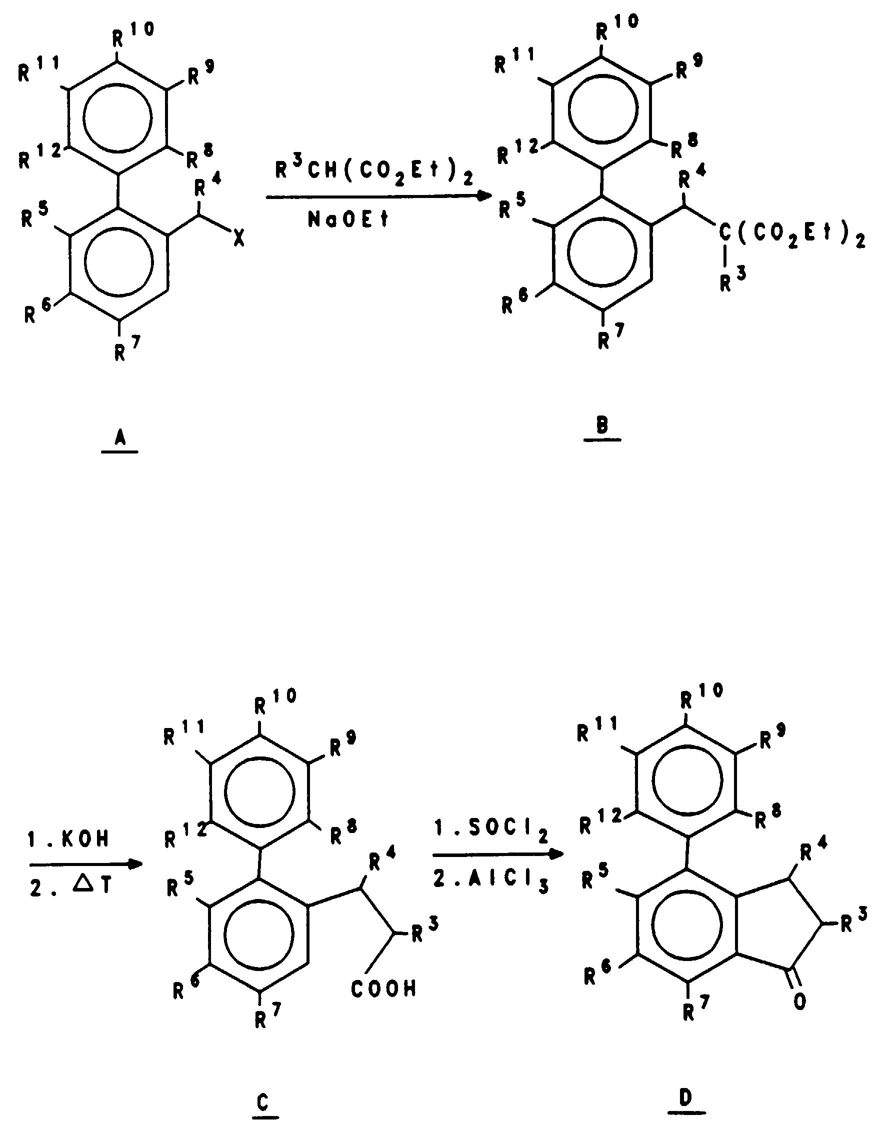 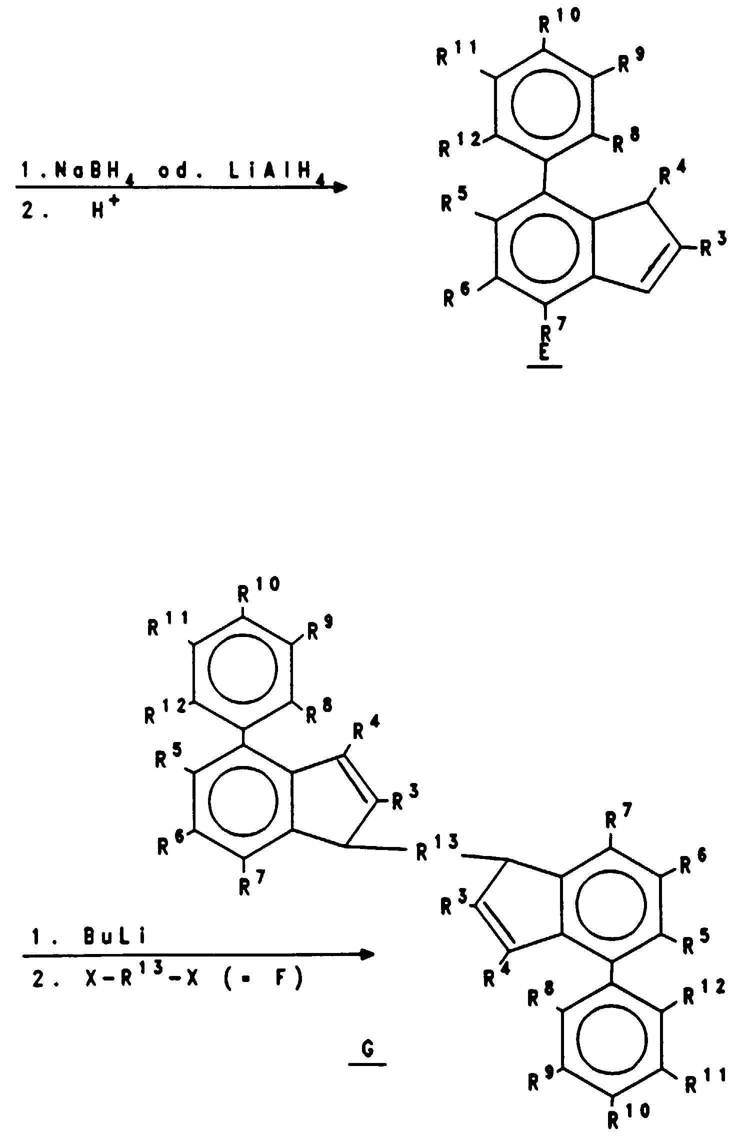
- X =: nukleophile Abgangsgruppe, beispielsweise Halogen oder Tosyl.

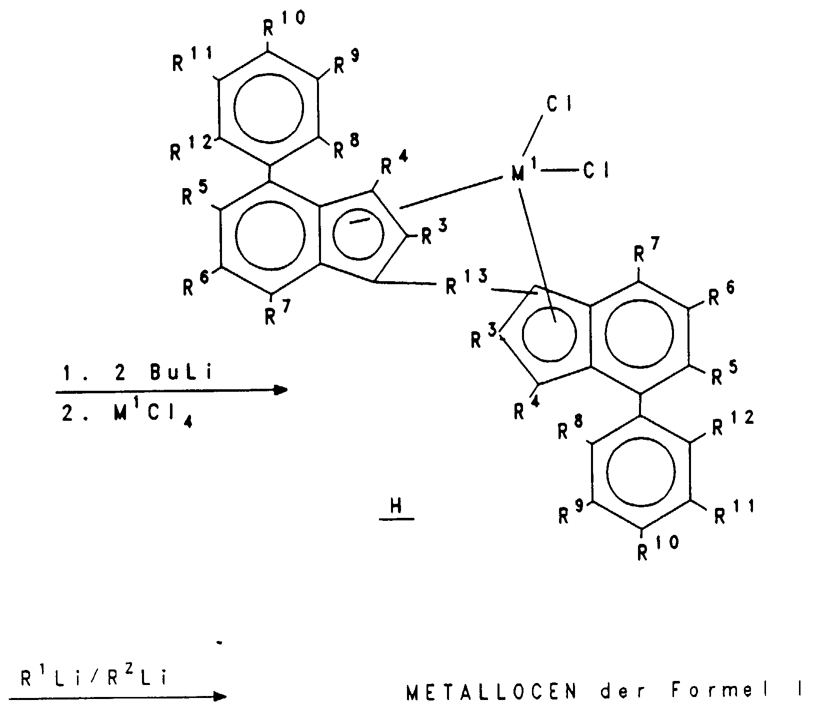

Die 2-Phenyl-benzylhalogenidderivate der Formel A sind im Handel erhältlich oder können nach literaturbekannten Methoden hergestellt werden.

Die Umsetzung zu den Verbindungen der Formel B erfolgt durch Reaktion mit substituierten Malonsäureestern unter basischen Bedingungen, wie beispielsweise in ethanolischen Lösungen von Natriumethanolat.

Die Verbindungen der Formel B werden mit Alkalihydroxiden wie Kaliumhydroxid oder Natriumhydroxid verseift und durch Behandeln der entstandenen Dicarbonsäuren mit Wärme zu den Verbindungen der Formel C decarboxyliert.

Der Ringschluß zu den entsprechenden Phenyl-1-indanonen der Formel D erfolgt durch Umsetzung mit Chlorierungsreagentien wie beispielsweise SOCl₂ zu den entsprechenden Säurechloriden und anschließender Cyclisierung mit einem Friedel-Crafts-Katalysator in einem inerten Solvent, wie z.B. AlCl₃ oder Polyphosphorsäure in Methylenchlorid oder CS₂.

Die Umsetzung zu den 7-Phenyl-indenderivaten der Formel E erfolgt durch Reduktion mit einem hydridübertragenden Reagenz, wie beispielsweise Natriumborhydrid oder Lithiumaluminiumhydrid oder Wasserstoff und einem entsprechenden Katalysator in einem inerten Lösungsmittel wie beispielsweise Diethylether oder Tetrahydrofuran zu den entsprechenden Alkoholen und Dehydratisierung der Alkohole unter sauren Bedingungen, wie beispielsaweise p-Toluolsulfonsäure oder einer wässrigen Mineralsäure oder durch Umsetzung mit wasserentziehenden Substanzen wie Magnesiumsulfat, wasserfreiem Kupfersulfat oder Molekularsiebe.

Die Herstellung der Ligandsysteme der Formel G und die Umsetzung zu den verbrückten chiralen Metallocenen der Formel H sowie die Isolierung der gewünschten racemischen Form ist im Prinzip bekannt. Hierzu wird das Phenylindenderivat der Formel E mit einer starken Base wie beispielsweise Butyllithium oder Kaliumhydrid in einem inerten Lösungsmittel deprotoniert und mit einem Reagenz der Formel F zu dem Ligandsystem der Formel G umgesetzt. Dieses wird anschließend mit zwei Äquivalenten einer starken Base wie beispielsweise Butyllithium oder Kaliumhydrid in einem inerten Lösungsmittel deprotoniert und mit dem entsprechenden Metalltetrahalogenid wie beispielsweise Zirkoniumtetrachlorid in einem geeigneten Lösemittel umgesetzt. Geeignete Lösemittel sind aliphatische oder aromatische Lösemittel, wie beispielsweise Hexan oder Toluol, etherische Lösemittel, wie beispielsweise Tetrahydrofuran oder Diethylether oder halogenierte Kohlenwasserstoffe, wie beispielsweise Methylenchlorid oder halogenierte aromatische Kohlenwasserstoffe wie beispielsweise o-Dichlorbenzol. Die Trennung der racemischen und der meso-Form erfolgt durch Extraktion oder Umkristallisation mit geeigneten Lösemitteln.

Die Derivatisierung zu den Metallocenen der Formel I kann beispielsweise durch Umsetzung mit Alkylierungsmitteln wie Methyllithium erfolgen.

Die erfindungsgemäßen Metallocene I sind hochaktive Katalysatorkomponenten für die Olefinpolymerisation. Die chiralen Metallocene werden bevorzugt als Racemat eingesetzt. Verwendet werden kann aber auch das reine Enantiomere in der (+)- oder (-)-Form. Mit den reinen Enantiomeren ist ein optisch aktives Polymer herstellbar. Abgetrennt werden sollte jedoch die meso-Form der Metallocene, da das polymerisationsaktive Zentrum (das Metallatom) in diesen Verbindungen wegen der Spiegelsymmetrie am Zentralmetallatom nicht mehr chiral ist und daher kein hochisotaktisches Polymeres erzeugen kann. Wird die meso-Form nicht abgetrennt, ensteht neben isotaktischen Polymeren auch ataktisches Polymer. Für bestimmte Anwendungen, beispielsweise weiche Formkörper, kann dies durchaus wünschenswert sein.

Erfindungsgemäß wird als Cokatalysator bevorzugt ein Aluminoxan der Formel lla für den linearen Typ und/oder der Formel llb für den cyclischen Typ verwendet, 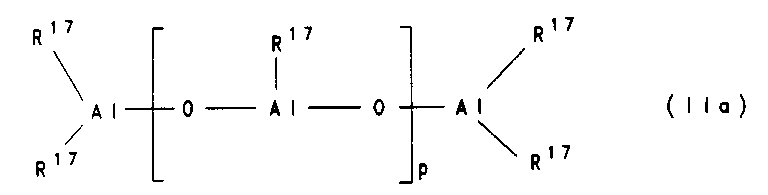 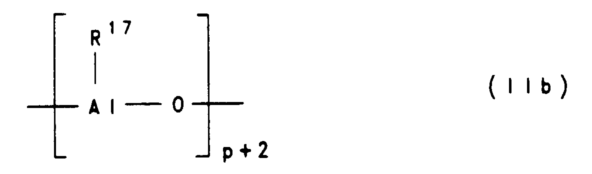 wobei in den Formeln lla und llb die Reste R¹⁷ gleich oder verschieden sein können und eine C₁-C₆- Alkylgrupppe, eine C₆-C₁₈-Arylgruppe, Benzyl oder Wasserstoff bedeuten, und p eine ganze Zahl von 2 bis 50, bevorzugt 10 bis 35 bedeutet.

Bevorzugt sind die Reste R¹⁷ gleich und bedeuten Methyl, Isobutyl, Phenyl oder Benzyl, besonders bevorzugt Methyl.

Sind die Reste R¹⁷ verschieden, so sind sie bevorzugt Methyl und Wasserstoff oder alternativ Methyl und Isobutyl, wobei Wasserstoff oder Isobutyl bevorzugt zu 0,01 - 40% (Zahl der Reste R¹⁷) enthalten sind.

Das Aluminoxan kann auf verschiedene Arten nach bekannten Verfahren hergestellt werden. Eine der Methoden ist beispielsweise, daß eine Aluminiumkohlenwasserstoffverbindung und/oder ein Hydridoaluminiumkohlenwasserstoffverbindung mit Wasser (gasförmig, fest, flüssig oder gebunden - beispielsweise als Kristallwasser) in einem inerten Lösungsmittel (wie beispielsweise Toluol) umgesetzt wird. Zur Herstellung eines Aluminoxans mit verschiedenen Resten R¹⁷ werden beispielsweise entsprechend der gewünschten Zusammensetzung zwei verschiedene Aluminiumtrialkyle mit Wasser umgesetzt.

Die genaue Struktur der Aluminoxane lla und llb ist nicht bekannt.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist möglich, das Metallocen vor dem Einsatz in der Polymerisationsreaktion mit einem Aluminoxan der Formel IIa und/oder IIb vorzuaktivieren. Dadurch wird die Polymerisationsaktivität deutlich erhöht und die Kornmorphologie verbessert. Die Voraktivierung der Übergangsmetallverbindung wird in Lösung vorgenommen. Bevorzugt wird dabei das Metallocen in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.%, jeweils bezogen auf die Gesamtlösungsmenge. Das Metallocen kann in der gleichen Konzentration eingesetzt werden, vorzugsweise wird es jedoch in einer Menge von 10⁻⁴ - 1 mol pro mol Aluminoxan eingesetzt. Die Voraktivierung beträgt 5 Minuten bis 60 Stunden, vorzugsweise 5 bis 60 Minuten. Man arbeitet bei einer Temperatur von - 78 bis 100°C, vorzugsweise 0 bis 70°C.

Mit Hilfe des Metallocens kann eine Vorpolymerisation erfolgen. Zur Vorpolymerisation wird bevorzugt das (oder eines der) in der Polymerisation eingesetzte(n) Olefin(e) verwendet.

Das Metallocen kann auch auf einen Träger aufgebracht werden. Geeignete Trägermaterialien sind beispielsweise Silikagele, Aluminiumoxide, festes Aluminoxan oder andere anorganische Trägermaterialien wie beispielsweise Magnesiumchlorid. Ein geeignetes Trägermaterial ist auch ein Polyolefinpulver in feinverteilter Form.

Vorzugsweise wird der Cokatalysator, d.h. die aluminiumorganische Verbindung, auf einen Träger wie beispielsweise Silikagele, Aluminiumoxide, festes Aluminoxan, andere anorganische Trägermaterialien oder auch ein Polyolefinpulver in feinverteilter Form aufgebracht und dann mit dem Metallocen umgesetzt.

Als anorganische Träger können Oxide eingesetzt werden, die flammenpyrolytisch durch Verbrennung von Element-Halogeniden in einer Knallgas-Flamme erzeugt wurden, oder als Kieselgele in bestimmten Korngrößen-Verteilungen und Kornformen herstellbar sind.

Die Herstellung des geträgerten Cokatalysators kann beispielsweise wie in EP 92 107 331.8 beschrieben in der folgenden Weise in einem Edelstahl-Reaktor in explosionsgeschützter Ausführung mit einem Umpumpsystem der Druckstufe 60 bar, mit Inertgasversorgung, Temperierung durch Mantelkühlung und zweitem Kühlkreislauf über einen Wärmetauscher am Umpumpsystem erfolgen. Das Umpumpsystem saugt den Reaktorinhalt über einen Anschluß im Reaktorboden mit einer Pumpe an und drückt ihn in einen Mischer und durch eine Steigleitung über einen Wärmetauscher in den Reaktor zurück. Der Mischer ist so gestaltet, daß sich in dem Zulauf ein verengter Rohrquerschnitt befindet, wo eine erhöhte Strömungsgeschwindigkeit entsteht, und in dessen Turbulenzzone axial und entgegen der Strömungsrichtung eine dünne Zuleitung geführt ist, durch welche - getaktet - jeweils eine definierte Menge Wasser unter 40 bar Argon eingespeist werden kann. Die Kontrolle der Reaktion erfolgt über einen Probennehmer am Umpumpkreislauf.

Im Prinzip sind jedoch auch andere Reaktoren geeignet.

Im vorstehend beschriebenen Reaktor mit 16 dm³ Volumen werden 5 dm³ Decan unter Inertbedingungen vorgelegt. 0,5 dm³ (=5,2 mol) Trimethylaluminium werden bei 25°C zugefügt. Danach werden 250 g Kieselgel SD 3216-30 (Grace AG), welche vorher bei 120°C in einem Argonfließbett getrocknet wurden, durch einen Feststofftrichter in den Reaktor eindosiert und mit Hilfe des Rührers und des Umpumpsystems homogen verteilt. Eine Gesamtmenge von 76,5 g Wasser wird in Portionen von 0,1 cm³ während 3,25 h jeweils alle 15 s in den Reaktor gegeben. Der Druck, herrührend vom Argon und den entwickelten Gasen, wird durch ein Druckregelventil konstant bei 10 bar gehalten. Nachdem alles Wasser eingebracht worden ist, wird das Umpumpsystem abgeschaltet und das Rühren noch 5 h bei 25°C fortgesetzt.

Der in dieser Weise hergestellte geträgerte Cokatalysator wird als eine 10 %ige Suspension in n-Decan eingesetzt. Der Aluminiumgehalt ist 1,06 mmol Al pro cm³ Suspension. Der isolierte Feststoff enthält 31 Gew.% Aluminium, das Suspensionsmittel enthält 0,1 Gew.% Aluminium.

Weitere Möglichkeiten der Herstellung eines geträgerten Cokatalysators sind in EP 92 107331.8 beschrieben.

Danach wird das erfindungsgemäße Metallocen auf den geträgerten Cokatalysator aufgebracht, indem das gelöste Metallocen mit dem geträgerten Cokatalysator gerührt wird. Das Lösemittel wird entfernt und durch einen Kohlenwasserstoff ersetzt, in dem sowohl Cokatalysator als auch das Metallocen unlöslich sind.

Die Reaktion zu dem geträgerten Katalysatorsystem erfolgt bei einer Temperatur von -20° bis +120°C, bevorzugt 0-100°C, besonders bevorzugt bei 15° bis 40°C. Das Metallocen wird mit dem geträgerten Cokatalysator in der Weise umgesetzt, daß der Cokatalysator als Suspension mit 1 bis 40 Gew%, bevorzugt mit 5 bis 20 Gew% in einem aliphatischen, inerten Suspensionsmittel wie n-Decan, Hexan, Heptan, Dieselöl mit einer Lösung des Metallocens in einem inerten Lösungsmittel wie Toluol, Hexan, Heptan, Dichlormethan oder mit dem feingemahlenen Feststoff des Metallocens zusammengebracht wird. Umgekehrt kann auch eine Lösung des Metallocens mit dem Feststoff des Cokatalysators umgesetzt werden.

Die Umsetzung erfolgt durch intensives Mischen, beispielsweise durch Verrühren bei einem molaren Al/M¹-Verhältnis von 100/1 bis 10000/1, bevorzugt von 100/1 bis 3000/1 sowie einer Reaktionszeit von 5 bis 120 Minuten, bevorzugt 10 bis 60 Minuten, besonders bevorzugt 10 bis 30 Minuten unter inerten Bedingungen.

Im Laufe der Reaktionszeit zur Herstellung des geträgerten Katalysatorsystems treten insbesondere bei der Verwendung der erfindungsgemäßen Metallocene mit Absorptionsmaxima im sichtbaren Bereich Veränderungen in der Farbe der Reaktionsmischung auf, an deren Verlauf sich der Fortgang der Reaktion verfolgen läßt.

Nach Ablauf der Reaktionszeit wird die überstehende Lösung abgetrennt, beispielsweise durch Filtration oder Dekantieren. Der zurückbleibende Feststoff wird 1- bis 5-mal mit einem inerten Suspensionsmittel wie Toluol, n-Decan, Hexan, Dieselöl, Dichlormethan zur Entfernung löslicher Bestandteile im gebildeten Katalysator, insbesondere zur Entfernung von nicht umgesetzten und damit löslichem Metallocen, gewaschen.

Das so hergestellte geträgerte Katalysatorsystem kann im Vakuum getrocknet als Pulver oder noch Lösemittel behaftet wieder resuspendiert und als Suspension in einem der vorgenannten inerten Suspensionsmittel in das Polymerisationssystem eindosiert werden.

Erfindungsgemäß können an Stelle oder neben eines Aluminoxans Verbindungen der Formeln R¹⁸ₓNH₄₋ₓBR¹⁹₄, R¹⁸ₓPH₄₋ₓBR¹⁹₄, R¹⁸₃CBR¹⁹₄, BR¹⁹₃ als geeignete Cokatalysatoren verwendet werden. In diesen Formeln bedeutet x eine Zahl von 1 bis 4, bevorzugt 3, die Reste R¹⁸ sind gleich oder verschieden, bevorzugt gleich, und bedeuten C₁-C₁₀-Alkyl, C₆-C₁₈-Aryl oder 2 Reste R¹⁸ bilden zusammen mit dem sie verbindenden Atom einen Ring, und die Reste R¹⁹ sind gleich oder verschieden, bevorzugt gleich, und stehen für C₆-C₁₈-Aryl, das durch Alkyl, Haloalkyl oder Fluor substituiert sein kann.
Insbesondere steht R¹⁸ für Ethyl, Propyl, Butyl oder Phenyl und R¹⁹ für Phenyl, Pentafluorphenyl, 3,5-Bistrifluormethylphenyl, Mesityl, Xylyl oder Tolyl (vgl. EP 277 003, EP 277 004 und EP 426 638).

Bei Verwendung der obengenannten Cokatalysatoren besteht der eigentliche (aktive) Polymerisationskatalysator aus dem Reaktionsprodukt von Metallocen und einer der genannten Verbindungen. Daher wird zunächst dieses Reaktionsprodukt bevorzugt außerhalb des Polymerisationsreaktors in einem separaten Schritt unter Verwendung eines geeigneten Lösemittels hergestellt.

Prinzipiell ist als Cokatalysator erfindungsgemäß jede Verbindung geeignet, die aufgrund ihrer Lewis-Acidität das neutrale Metallocen in ein Kation überführen und dieses stabilisieren kann ("labile Koordination"). Darüberhinaus soll der Cokatalysator oder das aus ihm gebildete Anion keine weiteren Reaktionen mit dem gebildeten Metallocenkation eingehen (vgl. EP 427 697).

Zur Entfernung von im Olefin vorhandenen Katalysatorgiften ist eine Reinigung mit einem Aluminiumalkyl, beispielsweise Trimethylaluminium oder Triethylaluminium vorteilhaft. Diese Reinigung kann sowohl im Polymerisationssystem selbst erfolgen, oder das Olefin wird vor der Zugabe in das Polymerisationssystem mit der Al-Verbindung in Kontakt gebracht und anschließend wieder abgetrennt.

Die Polymerisation oder Copolymerisation wird in bekannter Weise in Lösung, in Suspension oder in der Gasphase, kontinuierlich oder diskontinuierlich, ein- oder mehrstufig bei einer Temperatur von -60 bis 200°C, vorzugsweise 30 bis 80°C, besonders bevorzugt 50 bis 80°C, durchgeführt. Polymerisiert oder copolymerisiert werden Olefine der Formel R^{a}-CH=CH-R^{b}. In dieser Formel sind R^{a} und R^{b} gleich oder verschieden und bedeuten ein Wasserstoffatom, oder einen Alkylrest mit 1 bis 14 C-Atomen. R^{a} und R^{b} können jedoch auch mit den sie verbindenden C-Atomen einen Ring bilden. Beispiele für solche Olefine sind Ethylen, Propylen, 1-Buten, 1-Hexen, 4-Methyl-1-penten, 1-Octen, Norbornen oder Norbornadien. Insbesondere werden Propylen und Ethylen polymerisiert.

Als Molmassenregler und/oder zur Steigerung der Aktivität wird, falls erforderlich, Wasserstoff zugegeben. Der Gesamtdruck im Polymerisationssystem beträgt 0,5 bis 100 bar. Bevorzugt ist die Polymerisation in dem technisch besonders interessanten Druckbereich von 5 bis 64 bar.

Dabei wird das Metallocen in einer Konzentration, bezogen auf das Übergangsmetall von 10⁻³ bis 10⁻⁸, vorzugsweise 10⁻⁴ bis 10⁻⁷ mol Übergangsmetall pro dm³ Lösemittel bzw. pro dm³ Reaktorvolumen angewendet. Das Aluminoxan wird in einer Konzentration von 10⁻⁵ bis 10⁻¹ mol, vorzugsweise 10⁻⁴ bis 10⁻² mol pro dm³ Lösemittel bzw. pro dm³ Reaktorvolumen verwendet. Die anderen genannten Cokatalysatoren werden in etwa äquimolaren Mengen zum Metallocen verwendet. Prinzipiell sind aber auch höhere Konzentrationen möglich.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Hexan, Heptan, Isooctan, Cyclohexan, Methylcyclohexan, genannt. Weiterhin kann eine Benzin- bzw. hydrierte Dieselölfraktion benutzt werden. Brauchbar ist auch Toluol. Bevorzugt wird im flüssigen Monomeren polymerisiert.

Werden inerte Lösemittel verwendet, werden die Monomeren gasförmig oder flüssig zudosiert.

Die Dauer der Polymerisation ist beliebig, da das erfindungsgemäß zu verwendende Katalysatorsystem einen nur geringen zeitabhängigen Abfall der Polymerisationsaktivität zeigt.

Vor der Zugabe des Katalysators, insbesondere des geträgerten Katalysatorsystems (aus einem erfindungsgemäßen Metallocen und einem geträgerten Cokatalysator beziehungsweise aus einem erfindungsgemäßen Metallocen und einer aluminiumorganischen Verbindung auf einem Polyolefinpulver in feinverteilter Form), kann zusätzlich eine andere Aluminiumalkylverbindung wie beispielsweise Trimethylaluminium, Triethylaluminium, Triisobutylaluminium, Trioctylaluminium oder Isoprenylaluminium zur Inertisierung des Polymerisationssystems (beispielsweise zur Abtrennung vorhander Katalysatorgifte im Olefin) in den Reaktor gegeben werden. Diese wird in einer Konzentration von 100 bis 0,01 mmol Al pro kg Reaktorinhalt dem Polymerisationssystem zugesetzt. Bevorzugt werden Triisobutylaluminium und Triethylaluminium in einer Konzentration von 10 bis 0,1 mmol Al pro kg Reaktorinhalt. Dadurch kann bei der Synthese eines geträgerten Katalysatorsystems das molare Al/M¹-Verhältnis klein gewählt werden.

Grundsätzlich ist jedoch der Einsatz weiterer Substanzen zur Katalyse der Polymerisationsreaktion nicht erforderlich, d.h., die erfindungsgemäßen Systeme können als alleinige Katalysatoren für die Olefinpolymerisation verwendet werden.

Das erfindungsgemäße Verfahren zeichnet sich dadurch aus, daß die beschriebenen Metallocene im technisch besonders interessanten Temperaturbereich von 50 bis 80°C bei hohen Katalysatoraktivitäten Polymere mit sehr hoher Molmasse, im Fall prochiraler Monomere sehr hoher Molmasse und sehr hoher Stereotaktizität erzeugen.

Insbesondere zeichnen sich die erfindungsgemäßen Zirkonocene dadurch aus, daß im Fall der stereospezifischen Polymerisation prochiraler Olefine, beispielsweise von Propylen, Polymere mit hoher Isotaxie erhalten werden.

Insbesondere im Fall der isospezifischen Polymerisation von Propylen erhält man isotaktisches Polypropylen mit hohen isotaktischen Sequenzlängen und hohem Schmelzpunkt.

Darüber hinaus werden mit den erfindungsgemäß geträgerten Katalysatorsystemen Reaktorbeläge vermieden.

Die nachfolgenden Beispiele sollen die Erfindung näher erläutern.

Alle Glasgeräte wurden im Vakuum ausgeheizt und mit Argon gespült. Alle Operationen wurden unter Ausschluß von Feuchtigkeit und Sauerstoff in Schlenkgefäßen durchgeführt. Die verwendeten Lösemittel wurden unter Argon jeweils frisch über Na/K-Legierung destilliert und in Schlenk-Gefäßen aufbewahrt.

Die Ermittlung des Al/CH₃-Verhältnisses im Aluminoxan erfolgte durch Zersetzung der Probe mit H₂SO₄ und Bestimmung des Volumens der entstehenden Hydrolysegase unter Normalbedingungen sowie durch komplexometrische Titration des Aluminiums in der dann gelösten Probe nach Schwarzenbach.

Für die Beispiele Nr. 3 bis 5 mit der geträgerten Aluminiumverbindung (Methylaluminoxan auf Kieselgel), im folgenden "MAO auf SiO₂" genannt, wurde eine ca. 10 Gew.-% Suspension in n-Decan hergestellt, welche gemäß Aluminium-Bestimmung 60 mg Al/cm³ enthielt.

Für die Beispiele 26 bis 30 mit der geträgerten Aluminiumverbindung (Methylaluminoxan auf Kieselgel SD 3216-30/Grace), im folgenden "FMAO auf SiO₂" genannt, wurde ein lösungsmittelfreies Pulver verwendet, das 20 Gew.-% Aluminium im Feststoff enthielt.

Toluol lösliches Methylaluminoxan wurde für die Beispiele zur Suspensionspolymerisation und zur Massepolymerisation mit ungeträgertem Metallocen als 10 Gew.-%ige Toluol-Lösung eingesetzt und enthielt gemäß Aluminium-Bestimmung 36 mg Al/cm³. Der mittlere Oligomerisationsgrad gemäß Gefrierpunktserniedrigung in Benzol betrug n = 20. Für das Toluol lösliche Methylalumoxan wurde ein Verhältnis Al : CH₃ = 1 : 1,55 ermittelt.

Es bedeuten:
- VZ =: Viskositätszahl in cm³/g
- M_{w} =: Molmassengewichtsmittel in g/mol (ermittelt durch Gelpermeationschromatographie)
- M_{w}/Mₙ =: Molmassendispersität
- Schmp. =: Schmelzpunkt in °C (ermittelt mit DSC, 20°C/min Aufheiz-/Abkühlgeschwindigkeit)
- II =: Isotaktischer Index (II = mm + 1/2 mr, ermittelt durch ¹³C-NMR-Spektroskopie)
- MFI 230/5 SD =: Schmelzindex, gemessen nach DIN 53735; in dg/min Polymerschüttdichte in g/dm³.

### Synthese der in den Polymerisationsbeispielen verwendeten Metallocene I (die eingesetzten Edukte sind kommerziell erhältlich):

### A. rac-Dimethylsilyl-bis(2-methyl-4-phenyl-indenyl)zirkoniumdichlorid (5)

### 1. (±)-2-(2-Phenyl-benzyl)-propionsäure (1).

Zu 6,5 g (0,285 mol) Natrium in 160 cm³ H₂O-freiem EtOH wurden bei Raumtemperatur 48,6 g (0,279 mol) Diethylmethylmalonat zugetropft. Anschließend wurden 70,4 g (0,285 mol) 2-Phenyl-benzylbromid in 20 cm³ H₂O-freiem EtOH zugetropft und der Ansatz 3 h zum Rückfluß erhitzt. Das Lösemittel wurde abgezogen und der Rückstand mit 200 cm³ H₂O versetzt. Die organische Phase wurde abgetrennt, die wässrige Phase mit NaCl gesättigt und 2mal mit je 200 cm³ Et₂O extrahiert. Die mit den Extrakten vereinigte organische Phase wurde getrocknet (MgSO₄).

Der nach Abziehen des Lösemittels verbliebene Rückstand wurde in 500 cm³ EtOH und 50 cm³ H₂O aufgenommen und mit 56 g (1 mol) KOH versetzt. Die Reaktionsmischung wurde 4 h unter Rückfluß erhitzt. Das Lösemittel wurde im Vakuum abgezogen, der Rückstand in 500 cm³ H₂O aufgenommen und mit konzentrierter wässriger HCl bis pH 1 angesäuert. Der ausgefallene Niederschlag wurde abgesaugt und am Kugelrohr 30 min unter starkem Aufschäumen auf 250°C erhitzt. Man erhielt 58,3 g (85%) 1 als zähflüssiges Öl.
¹H-NMR (100 MHz, CDCl₃): 11,7 (s, 1H, COOH), 7,1-7,5 (m, 9H, arom. H), 2,3 - 3,2 (m, 3H, CH u. CH₂), 0,9 (d, 3H, CH₃).

### 2. (±)-2-Methyl-4-phenyl-indan-1-on (2)

Eine Lösung von 58 g (0,242 mol) 1 in 60 cm³ (0,83 mol) Thionylchlorid wurde 18 h bei Raumtemperatur gerührt. Überschüssiges Thionylchlorid wurde bei 10 mbar entfernt und der ölige Rückstand durch mehrmaliges Lösen in je 100 cm³ Toluol und Abziehen im Vakuum von anhaftenden Resten Thionylchlorid befreit.

Das Säurechlorid wurde in 150 cm³ Toluol aufgenommen und bei 10°C zu einer Suspension von 48 g (0,363 mol) AlCl₃ in 400 cm³ Toluol getropft. Nach vollständiger Zugabe wurde das Gemisch noch 3 h unter Rückfluß erhitzt. Die Reaktionsmischung wurde auf 500 g Eis gegossen und mit konzentrierter wässriger HCl bis pH 1 angesäuert. Die organische Phase wurde abgetrennt und die wässrige Phase 3mal mit je 100 cm³ Et₂O nachextrahiert. Die vereinigten organischen Phasen wurden mit gesättigter wässriger NaHCO₃-, gesättigter wässriger NaCl-Lösung gewaschen und danach getrocknet (MgSO₄). Es wurden 50,4 g (93%) 2 erhalten, das ohne weitere Reinigung weiter umgesetzt wurde.
¹H-NMR (100 MHz, CDCl₃): 7,2-7,8 (m, 8H, arom. H), 3,3 (dd, 1H, β-H), 2,5 - 2,9 (m, 2H, α- und β-H), 1,3 (d, 3H, CH₃).

### 3. 2-Methyl-7-phenyl-inden (3)

50 g (0,226 mol) 2 wurden in 450 cm³ THF/MeOH (2:1) gelöst und bei 0°C unter Rühren portionsweise mit 12,8 g (0,34 mol) Natriumborhydrid versetzt und 18 h weiter gerührt. Die Reaktionsmischung wurde auf Eis gegossen, mit konzentrierter HCl bis pH 1 versetzt und mehrmals mit Et₂O extrahiert. Die vereinigten organischen Phasen wurden mit gesättigter wässriger NaHCO₃-, NaCl-Lösung gewaschen und danach getrocknet (MgSO₄). Das Lösemittel wurde im Vakuum entfernt und das Rohprodukt ohne weitere Reinigung in 1 dm³ Toluol aufgenommen, mit 2 g p-Toluolsulfonsäure versetzt und 2 h zum Rückfluß erhitzt. Die Reaktionsmischung wurde mit 200 cm³ gesättigter wässriger NaHCO₃-Lösung gewaschen und das Lösemittel im Vakuum entfernt. Das Rohprodukt wurde durch Filtration über 500 g Kieselgel (Hexan/CH₂Cl₂) gereinigt. Es wurden 42 g (90%) 3 als farbloses Öl erhalten.
¹H-NMR (100 MHz, CDCl₃): 7,0-7,6 (m, 8H, arom. H), 6,5 (m, 1H, H- C(3)), 3,4 (s, 2H, CH₂), 2,1 (s, 3H, CH₃).

### 4. Dimethylbis(2-methyl-4-phenyl-indenyl)silan (4)

Eine Lösung von 15 g (72,7 mmol) 3 in 200 cm³ H₂O- und O₂-freiem Toluol und 10 cm³ H₂O- und O₂-freiem THF wurden bei Raumtemperatur unter Argon mit 29 cm³ (73 mmol) einer 2,5 M Lösung von Butyllithium in Hexan versetzt und 1 h auf 80°C erhitzt. Anschließend wurde der Ansatz auf 0°C gekühlt und mit 4,7 g (36,4 mmol) Dimethyldichlorsilan versetzt. Das Gemisch wurde 1 h auf 80°C erhitzt und anschließend auf 100 cm³ H₂O gegossen. Es wurde mehrmals mit Et₂O extrahiert und die vereinigten organischen Phasen getrocknet (MgSO₄). Das nach Abziehen des Lösemittels im Vakuum verbliebene Rohprodukt wurde an 300 g Kieselgel (Hexan/CH₂Cl₂) chromtographiert. Es wurden 12,0 g (70%) 4 erhalten.
¹H-NMR (100 MHz, CDCl₃): 7,10 - 7,70 (m, 16H, arom. H), 6,80 (m, 2H, H-C(3)), 3,80 (s, 2H, H-C(1)), 2,20 (m, 6H, CH₃), -0,20 (m, 6H, CH₃Si).

### 5.rac-Dimethylsilylbis(2-methyl-4-phenyl-indenyl)zirkoniumdichlorid (5)

Eine Lösung von 6,0 g (12,9 mmol) 4 in 100 cm³ H₂O- und O₂-freiem Toluol wurde unter Argon bei Raumtemperatur mit 10,6 cm³ (26 mmol) einer 2,5 M Lösung von Butyllithium in Hexan versetzt und 3 h zum Rückfluß erhitzt. Anschließend wurde die Suspension des Dilithiosalzes auf -25°C abgekühlt und mit 3,2 g (13,6 mmol) Zirkoniumtetrachlorid versetzt. Man erwärmte den Ansatz innerhalb 1 h auf Raumtemperatur, rührte ihn noch 1 h und filtrierte dann über eine G3-Fritte. Der Rückstand wurde mit 50 cm³ Toluol extrahiert und die vereinigten Filtrate im Vakuum einer Ölpumpe vom Lösemittel befreit. Man erhielt 9,0 g des Metallocens als Mischung der racemischen und der meso-Form im Verhältnis 1:1 in Form eines gelben Pulvers. Das reine Racemat (5) konnte durch mehrfaches Verrühren der Rohmischung mit je 20 cm³ Methylenchlorid gewonnen werden, wobei das Racemat als gelbes Kristallpulver zurückblieb und die meso-Form ausgewaschen wurde. Man erhielt 2,74 g (33%) des reinen Racemats.
¹H-NMR (300 MHz, CDCl₃): 7,0 - 7,7 (m, 16H, arom. H), 6,9 (s, 2H, H- C(3)), 2,2 (s, 6H, CH₃), 1,3 (m, 6H, CH₃Si).
Molmasse: 626 M⁺, korrektes Zerfallsmuster.

### Beispiel B

### rac-Methylphenylsilandiylbis-(2-methyl-4-phenylindenyl)zirkoniumdichlorid (7)

### 1. Methylphenylbis-(2-methyl-4-phenylindenyl)silan (6)

Eine Lsg. von 10,3 g (50 mmol) 3 in 90 ml H₂O- und O₂-freiem Toluol und 10 ml H₂O- und O₂-freiem THF wurden bei Raumtemperatur unter Argon mit 21 ml (52 mmol) eine 2,5 M Lsg. von Butyllithium in Hexan versetzt und 1 h auf 80°C erhitzt. Anschließend wurde auf 0°C gekühlt und mit 4,8 g (25 mmol) Methylphenyldichlorsilan versetzt und über Nacht bei RT weitergerührt. Das ausgefallene LiCl wurde durch Filtration abgetrennt und das nach Abziehen des Lösemittels im Vakuum verbleibende Rohprodukt an 300 g Kieselgel (Hexan/CH₂Cl₂ 9:1) chromatographiert. Es wurden 4,6 g (35%) 6 erhalten.
¹H-NMR (100 MHz, CDCl₃): 7,0 - 7,8 (m, 16H, arom. H), 6,9 (m, 2H, H- C(3)), 3,9 (m, 2H, H-C(1)), 2,3 (m, 6H, CH₃), -0,1(s, 3H, CH₃Si).

### 2. rac-Methylphenylsilandiylbis(2-methyl-4-phenylindenyl)zirkoniumdichlorid (7)

2,3 g (4,4 mmol) 6 in 25 ml H₂O- und O₂-freiem Toluol wurden bei Raumtemperatur unter Argon mit 3,6 ml (8,9 mmol) einer 2,5 M Lsg. von Butyllithium in Hexan versetzt und 3h auf 80°C erhitzt. Anschließend wurde die Suspension des Dilithiosalzes auf -30°C abgekühlt und mit 1,1 g (4,5 mmol) Zirkoniumtetrachlorid versetzt. Man erwärmte innerhalb 1 h auf Raumtemperatur, rührte noch 1 h nach. Nach Filtration über eine G3-Fritte wurde das Lösemittel des Filtrats entfernt und der Rückstand aus 10 ml Methylenchlorid kristallisiert. Man erhielt 0,2 g der racemischen Form von 7 als orange Kristalle.
¹H-NMR (100 MHz, CDCl₃): 7,0 - 8,2 (m, 21H, arom. H), 6,9 (m, 2H, H-C(3)), 2,4 (s, 3H, CH₃), 2,0 (s, 3H, CH₃), 1,3 (s, 3H, CH₃Si). Massenspektrum: 690 M⁺, korrektes Zerfallsmuster.

### Beispiel C

### rac-Dimethylsilandiylbis(4-phenylindenyl)zirkoniumdichlorid (12)

### 1. 3-(2-Phenyl-phenyl)propionsäure (8)

Zu 14 g (0,61 mmol) Natrium in 400 cm³ H₂O-freiem EtOH wurden bei Raumtemperatur 93 cm³ (0,61 mmol) Malonsäurediethylester gelöst in 50 cm³ H₂O-freiem EtOH zugetropft. Anschließend wurden 150 g (0,61 mmol) 2-Phenylbenzylbromid in 200 cm³ H₂O-freiem EtOH zugetropft und 3 h zum Rückfluß erhitzt. Bei Raumtemperatur wurden 102 g (1 ,83 mol) KOH gelöst in 150 cm³ H₂O zugesetzt und weitere 4 h zum Rückfluß erhitzt. Die Lösemittel wurden im Vakuum entfernt, der Rückstand bis zur vollständigen Lösung mit H₂O versetzt und mit konzentrierter wässr. HCl bis pH 1 angesäuert. Der ausgefallene Niederschlag wurde abgesaugt, getrocknet und 1 h auf 130°C erhitzt. Man erhielt 112 g (81%) 8 als zähflüssiges Öl.
¹H-NMR (100 MHz, CDCl₃): 9,1 (s, 1H, COOH), 6,9 - 7,5 (m, 9H, arom. H), 2,3 - 3,0 (m, 4H, 2 CH₂).

### 2. 4-Phenyl-1-indanon (9)

Eine Lösung von 102 g (0,45 mol) 8 in 37 cm³ (0,5 mol) Thionylchlorid wurde 18 h bei Raumtemperatur gerührt. Überschüssiges Thionylchlorid wurde bei 10 mbar entfernt und der ölige Rückstand durch mehrmaliges Lösen in je 100 cm³ Toluol und Abziehen im Vakuum von anhaftenden Resten Thionylchlorid befreit.

Das Säurechlorid wurde in 200 cm³ Toluol aufgenommen und bei 10°C zu einer Suspension von 72 g (0,54 mol) AlCl₃ in 1000 cm³ Toluol getropft und 1 h auf 80°C erhitzt. Die Reaktionsmischung wurde auf 1000 g Eis gegossen und mit konz. wässr. HCl bis pH 1 angesäuert. Die organische Phase wurde abgetrennt und die wässr. Phase 3mal mit je 200 cm³ Et₂O nachextrahiert. Die vereinigten organischen Phasen wurden mit gesättigter wässr. NaHCO₃-, gesättigter wässr. NaCl-Lösung gewaschen und anschließend getrocknet (MgSO₄). Es wurden 96 g (96%) 9 erhalten, das ohne weitere Reinigung weiter umgesetzt wurde.
¹H-NMR (100 MHz, CDCl₃): 6,9 - 7,5 (m, 8H, arom. H), 2,5 - 3,4 (m, 4H, 2 CH₂).

### 3. 7-Phenyl-inden (10)

Eine Lösung von 86 g (0,41 mol) 9 in 300 cm³ THF/Methanol 2:1 wurde bei 0°C portionsweise mit 23 g (0,62 mol ) NaBH₄ versetzt und 18 h bei Raumtemperatur gerührt. Die Reaktionsmischung wurde auf 300 g Eis gegossen, mit konz. wässr. HCl bis pH 1 versetzt und mehrmals mit Et₂O extrahiert. Die vereinigten organischen Phasen wurden mit gesättigter wässr. NaHCO₃-, gesättigter wässr. NaCl-Lösung gewaschen, getrocknet (MgSO₄) und im Vakuum vom Lösemittel befreit.

Das Rohprodukt wurde in 1000 cm³ Toluol aufgenommen, mit 4,5 g p-Toluolsulfonsäure versetzt und 2 h am Wasserabscheider zum Rückfluß erhitzt. Die Reaktionsmischung wurde 3mal mit 250 cm³ gesättigter wässr. NaHCO₃-Lösung gewaschen und das Lösemittel im Vakuum entfernt. Nach Destillation bei 0,1 mbar wurde bei 96-108°C 33 g (41 %) 10 als farbloses Öl erhalten.
¹H-NMR (100 MHz, CDCl₃): 7,1 - 7,7 (m, 8H, arom. H), 6,9 und 6,5 (2m, 2H, CH), 3,5 (m, 2H, CH₂).

### 4. Dimethylbis(4-phenylindenyl)silan (11)

Eine Lösung von 10 g (50 mmol) 10 in 100 cm³ H₂O- und O₂-freiem Toluol und 5 ml H₂O- und O₂-freiem THF wurden bei Raumtemperatur mit 18,7 cm³ (50 mmol) einer 20%igen Lösung von Butyllithium in Toluol versetzt und 2 h auf 80°C erhitzt. Anschließend wurde die gelbe Suspension auf 0°C gekühlt und mit 3,2 g (25 mmol) Dimethyldichlorsilan versetzt. Die Reaktionsmischung wurde noch 1 h auf 80°C erhitzt und anschließend mit 50 cm³ H₂O gewaschen. Das Lösemittel wurde im Vakuum entfernt und der Rückstand aus Heptan bei - 20°C umkristallisiert. Es wurden 6,7 g (62%) 11 als farblose Kristalle (Schmp. 109-110°C) erhalten.
¹H-NMR (100 MHz, CDCl₃): 7,0 - 7,7 (m, 18H, arom. H und H-C(3)), 6,8 (dd, 2H, H-C(2)), 3,8 (m, 2H, H-C(1)), -0,2, (s, 6H, CH₃Si).

### 5. rac-Dimethylsilandiylbis(4-phenylindenyl)zirkoniumdichlorid (12)

Eine Lösung von 6,6 g (16 mmol) 11 in 70 cm³ H₂O- und O₂-freiem Et₂O wurden unter Argon bei Raumtemperatur mit 12 cm³ (32 mmol) einer 20%igen Lösung von Butyllithium in Toluol versetzt und anschließend 3 h zum Rückfluß erhitzt. Das Lösemittel wurde im Vakuum entfernt und der Rückstand mit 50 ml H₂O- und O₂-freiem Hexan über eine G3-Schlenkfritte filtriert, mit 50 ml H₂O- und O₂-freiem Hexan nachgewaschen und getrocknet (0,1 mbar, RT).

Das Dilithiosalz wurde bei -78°C zu einer Suspension von 3,6 g (16 mmol) Zirkoniumtetrachlorid in 80 cm³ Methylenchlorid gegeben und im Verlauf von 18 h unter magn. Rühren auf Raumtemperatur erwärmt. Der Ansatz wurde über eine G3-Fritte filtriert und der Rückstand portionsweise mit insgesamt 200 cm³ Methylenchlorid nachextrahiert. Die vereinigten Filtrate wurden im Vakuum vom Lösemitel befreit und aus Methylenchlorid/Hexan (1:1) umkristallisiert. Es wurden 5,6 g der racemischen und der meso-Form im Verhältnis 1:1 erhalten. Durch erneutes Umkristallisieren aus Methylenchlorid wurde der racemische Komplex in Form gelber Kristalle erhalten.
¹H-NMR (100 MHz, CDCl₃): 7,0 - 7,8 (m, 22 H, arom. H und H-C(3)), 6,1 (d, 2H, H-C(2)), 1,1 (s, 6H, CH₃Si). Massenspektrum: 598 M⁺, korrektes Zerfallsmuster.

### Beispiel D

### rac-Dimethylsilandiylbis(2-ethyl-4-phenylindenyl)zirkoniumdichlorid (17)

### 1. (±)-2-(2-Phenylbenzyl)-buttersäure (13)

Zu 23 g (1 mol) Natrium in 400 cm³ H₂O-freiem EtOH wurden bei Raumtemperatur 188 g (1 mol) Ethyl-malonsäurediethylester gelöst in 100 cm³ H₂O-freiem EtOH zugetropft. Anschließend wurden 247 g (1 mol) 2-Phenylbenzylbromid in 300 cm³ H₂O-freiem EtOH zugetropft und 3 h zum Rückfluß erhitzt. Bei Raumtemperatur wurden 170 g (3 mol) KOH gelöst in 300 cm³ H₂O zugesetzt und weitere 4 h zum Rückfluß erhitzt. Das Lösemittel wurden im Vakuum entfernt, der Rückstand bis zur vollständigen Lösung mit H₂O versetzt und anschließend mit konzentrierter wässr. HCl bis pH 1 angesäuert. Der ausgefallene Niederschlag wurde abgesaugt, getrocknet und 1 h auf 130°C erhitzt. Man erhielt 236 g (93%) 13 als zähflüssiges Öl.
¹H-NMR (100 MHz, CDCl₃): 10,3 (s, 1H, COOH), 7,0 - 7,3 (m, 9 H, arom. H), 2,5 - 3,0 (m, 3H, CH und CH₂), 1,5 - 1,9 (m, 2H, CH₂), 0,9 (t, 3H, CH₃).

### 2. (±)-2-Ethyl-4-phenyl-1-indanon (14)

Eine Lösung von 236 g (0,93 mol) 13 in 81 cm³ (1,2 mol) Thionylchlorid wurde 18 h bei Raumtemperatur gerührt. Überschüssiges Thionylchlorid wurde bei 10 mbar entfernt und der ölige Rückstand durch mehrmaliges Lösen in je 200 cm³ Toluol und Abziehen im Vakuum von anhaftenden Resten Thionylchlorid befreit.

Das Säurechlorid wurde in 400 cm³ Toluol aufgenommen und bei 10°C zu einer Suspension von 133 g (1,0 mol) AlCl₃ in 2000 cm³ Toluol getropft und 1 h auf 80°C erhitzt. Die Reaktionsmischung wurde auf 2000 g Eis gegossen und mit konz. wässr. HCl bis pH 1 angesäuert. Die organische Phase wurde abgetrennt und die wässr. Phase 3mal mit je 200 cm³ Et₂O nachextrahiert. Die vereinigten organischen Phasen wurden mit gesättigter wässr. NaHCO₃-, gesättigter wässr. NaCl-Lösung gewaschen und anschließend getrocknet (MgSO₄).

Es wurden 187 g (85%) 14 erhalten, das ohne weitere Reinigung weiter umgesetzt wurde.
¹H-NMR (100 MHz, CDCl₃): 7,0 - 7,8 (m, 8 H, arom. H), 3,1 - 3,4 (m, 1H, H-C(3)), 2,5 - 2,9 (m, 2H, H-C(2) und H-C(3)), 1,3 - 2,0 (m, 2H, CH₂), 0,9 (t, 3H, CH₃).

### 3. 2-Ethyl-7-phenylinden (15)

Zu einer Lösung von 50 g (0,21 mol) 14 in 600 cm³ THF/Methanol 2:1 wurde bei 0°C portionsweise mit 8 g (0,21 mol) NaBH₄ versetzt und 18 h bei Raumtemperatur gerührt. Die Reaktionsmischung wurde auf 600 g Eis gegossen, mit konz. wässr. HCl bis pH 1 versetzt und mehrmals mit Et₂O extrahiert. Die vereinigten organischen Phasen wurden mit gesättigter wässr. NaHCO₃-, gesättigter wässr. NaCl-Lösung gewaschen und anschließend getrocknet (MgSO₄).

Das Rohprodukt wurde in 1000 cm³ Toluol aufgenommen, mit 4,5 g p-Toluolsulfonsäure versetzt und 2 h am Wasserabscheider zum Rückfluß erhitzt. Die Reaktionsmischung wurde 3mal mit 250 cm³ gesättigter wässr. NaHCO₃-Lösung gewaschen und das Lösemittel im Vakuum entfernt. Nach Destillation bei 0,1 mbar wurden bei 135°C 33 g (72%) 15 als farbloses Öl erhalten.
¹H-NMR (100 MHz, CDCl₃): 7,0 - 7,5 (m, 8H, arom. H), 6,5 (m, 1H, CH), 3,2 (m, 2H, CH₂), 2,5 (dq, 2H, CH₂), 1,1 (t, 3H, CH₃).

### 4. Dimethylbis(2-ethyl-4-phenylindenyl)silan (16)

Eine Lösung von 17 g (77 mmol) 15 in 160 cm³ H₂O- und O₂-freiem Toluol und 8 ml H₂O- und O₂-freiem THF wurden bei Raumtemperatur mit 29 cm³ (77 mmol) einer 20%igen Lösung von Butyllithium in Toluol versetzt und 2 h auf 80°C erhitzt. Anschließend wurde die gelbe Suspension auf 0°C gekühlt und mit 5 g (38 mmol) Dimethyldichlorsilan versetzt. Die Reaktionsmischung wurde noch 1 h auf 80°C erhitzt und anschließend mit 100 cm³ H₂O gewaschen. Das Lösemittel wurde im Vakuum entfernt und der Rückstand durch Chromatographie an 200 g Kieselgel (Hexan/Methylenchlorid 9:1) gereinigt. Es wurden 9 g (47%) 16 als zähflüssiges Öl erhalten.
¹H-NMR (100 MHz, CDCl₃): 6,9 - 7,4 (m, 16H, arom. H), 6,5 (m, 2H, H-C(3)), 3,7 (m, 2H, H-C(1)), 2,4 (m, 4H, CH₂), 1,1 (t, 6H, CH₃), -0,1, (s, 6H, CH₃Si).

### 5. rac-Dimethylsilandiylbis(2-ethyl-4-phenylindenyl)zirkoniumdichlorid (17)

Eine Lösung von 5,6 g (11 mmol) 16 in 50 cm³ H₂O- und O₂-freiem Et₂O wurden unter Argon bei Raumtemperatur mit 8,4 cm³ einer 20%igen Lösung von Butyllithium in Toluol versetzt und anschließend 3 h zum Rückfluß erhitzt. Das Lösemittel wurde im Vakuum entfernt und der Rückstand mit 50 ml H₂O- und O₂-freiem Hexan über eine G3-Schlenkfritte filtriert, mit 50 ml H₂O- und O₂-freiem Hexan nachgewaschen und getrocknet (0,1 mbar, RT).

Das Dilithiosalz wurde bei -78°C zu einer Suspension von 2,5 g (11 mmol) Zirkoniumtetrachlorid in 50 cm³ Methylenchlorid gegeben und im Verlauf von 18 h unter magn. Rühren auf Raumtemperatur erwärmt. Der Ansatz wurde über eine G3-Fritte filtriert und der Rückstand portionsweise mit insgesamt 100 cm³ Methylenchlorid nachextrahiert. Die vereinigten Filtrate wurden im Vakuum vom Lösemitel befreit und aus Toluol/Hexan (1:1) umkristallisiert. Es wurden 2 g (27%) der racemischen und der meso-Form im Verhältnis 1:1 erhalten. Durch erneutes Umkristallisieren aus Toluol wurde der racemische Komplex 17 in Form gelber Kristalle erhalten.
¹H-NMR (100 MHz, CDCl₃): 6,8 - 7,7 (m, 16 H, arom. H), 6,6 (m, 2H, H-C(3)), 2,3-3,9 (m, 4H, CH₂), 1,0 - 1,4 (m, 12H, CH₃ und CH₃Si). Massenspektrum: 654 M⁺, korrektes Zerfallsmuster.

### Beispiel E

### rac-Dimethylsilandiylbis(2-methyl-4-(1-naphthyl)indenyl)zirkoniumdichlorid (24)

### 1. 2-(1-Naphthyl)-toluol (18)

13,9 g (0,57 mol) Magnesium-Späne wurden mit 150 ml H₂O-freiem Et₂O überschichtet und die Grignard-Reaktion mit 5 g 2-Bromtoluol und einigen Körnchen Jod zum Anspringen gebracht. Anschließend wurden 93 g (0,57 mol) 1-Bromtoluol in 450 ml H₂O-freiem Et₂O so zugetropft, daß die Reaktionsmischung am Sieden gehalten wurde. Nach vollständiger Zugabe wurde noch solange zum Sieden erhitzt, bis das Magnesium vollständig umgesetzt war.

Die Grignard-Lösung wurde anschließend zu einer Lösung von 118 g (0,57 mol) 1-Bromnaphthalin und 3,5 g Bis(triphenylphosphin)nickeldichlorid in 800 cm³ Toluol getropft, so daß die Innentemp 50°C nicht überstieg. Anschließend wurde noch 3 h zum Rückfluß erhitzt, mit 500 ml 10%iger wässr. HCI versetzt, die Phasen getrennt und die organische Phase im Vakuum vom Lösemittel befreit. Nach Filtration über Kieselgel (Hexan) wurden 115 g (92%) 18 als farbloses Öl erhalten.
¹H-NMR (100 MHz, CDCl₃): 7,2 - 8,0 (m, 11H, arom. H), 2,0 (s, 3H, CH₃).

### 2. 2-(1-Naphthyl)-benzylbromid (19)

114 g (0,52 mol) 18 und 103 g (0,58 mol) N-Bromsuccinimid wurden bei Raumtemperatur in 2000 cm³ Tetrachlorkohlenstoff gelöst, mit 3 g Azobisisobutyronitril versetzt und 4 h zum Rückfluß erhitzt. Das ausgefallene Succinimid wurde abfiltriert, das Lösemittel im Vakuum entfernt und der Rückstand durch Filtration über 1000 g Kieselgel (Hexan/Methylenchlorid 9:1) gereinigt. Es wurden 141 g (82%) 19 als farbloses, tränenreizendes Öl erhalten.
¹H-NMR (100 MHz, CDCl₃): 7,1 - 8,0 (m, 11H, arom. H), 4,2 (q, 2H, CH₂Br).

### 3.(±)-2-(2-(1-naphthyl)benzyl)-propionsäure (20)

Zu 10 g (0,43 mmol) Natrium in 100 cm³ H₂O-freiem EtOH wurden bei Raumtemperatur 75 g (0,43 mmol) Methyl-malonsäurediethylester gelöst in 50 cm³ H₂O-freiem EtOH zugetropft. Anschließend wurden 140 g (0,43 mmol) 2-Phenylbenzylbromid in 200 cm³ H₂O-freiem EtOH zugetropft und 3 h zum Rückfluß erhitzt. Bei Raumtemperatur wurden 85 g (1,3 mol) KOH gelöst in 100 cm³ H₂O zugesetzt und weitere 4 h zum Rückfluß erhitzt. Die Lösemittel wurden im Vakuum entfernt, der Rückstand bis zur vollständigen Lösung mit H₂O versetzt und mit konzentrierter wässr. HCl bis pH 1 angesäuert. Der ausgefallene Niederschlag wurde abgesaugt, getrocknet und 1 h auf 130°C erhitzt. Man erhielt 96 g (77%) 20 als zähflüssiges Öl.
¹H-NMR (100 MHz, CDCl₃): 10,1 (s, 1H, COOH), 6,9 - 8,0 (m, 11H, arom. H), 2,3 - 3,0 (m, 3H, CH₂ und CH), 0,8 (d, 3H, CH₃).

### 4. (±)-2-Methyl-4-(1-naphthyl)-1-indanon (21)

Eine Lösung von 96 g (0,33 mol) 20 in 37 cm³ (0,5 mol) Thionylchlorid wurde 18 h bei Raumtemperatur gerührt. Überschüssiges Thionylchlorid wurde bei 10 mbar entfernt und der ölige Rückstand durch mehrmaliges Lösen in je 100 cm³ Toluol und Abziehen im Vakuum von anhaftenden Resten Thionylchlorid befreit.

Das Säurechlorid wurde in 200 cm³ Toluol aufgenommen und bei 10°C zu einer Suspension von 44 g (0,33 mol) AlCl₃ in 1000 cm³ Toluol getropft und 3 h auf 80°C erhitzt. Die Reaktionsmischung wurde auf 1000 g Eis gegossen und mit konz. wässr. HCl bis pH 1 angesäuert. Die organische Phase wurde abgetrennt und die wässr. Phase 3mal mit je 200 cm³ Methylenchlorid nachextrahiert. Die vereinigten organischen Phasen wurden mit gesättigter wässr. NaHCO₃-, gesättigter wässr. NaCl-Lösung gewaschen und anschließend getrocknet (MgSO₄). Nach Chromatographie an 1000 g Kieselgel (Hexan/Methylenchlorid) wurden 12 g (13%) 21 erhalten.
¹H-NMR (100 MHz, CDCl₃): 7,3 - 8,0 (m, 10H, arom. H), 2,2 - 3,2 (m, 3H, CH₂ und CH), 1,2 (d, 3H, CH₃).

### 5. 2-Methyl-7-(1-naphthyl)inden (22)

Zu einer Lösung von 12 g (44 mmol) 21 in 100 cm³ THF/Methanol 2:1 wurden bei 0°C 1,3 g (33 mmol) NaBH₄ zugesetzt und 18 h bei Raumtemperatur gerührt. Die Reaktionsmischung wurde auf 100 g Eis gegossen, mit konz. wässr. HCl bis pH 1 versetzt und mehrmals mit Et₂O extrahiert. Die vereinigten organischen Phasen wurden mit gesättigter wässr. NaHCO₃-, gesättigter wässr. NaCl-Lösung gewaschen und anschließend getrocknet (MgSO₄).

Das Rohprodukt wurde in 200 cm³ Toluol aufgenommen, mit 0,5 g p-Toluolsulfonsäure versetzt und 2 h am Wasserabscheider zum Rückfluß erhitzt. Die Reaktionsmischung wurde 3mal mit 50 cm³ gesättigter wässr. NaHCO₃-Lösung gewaschen und das Lösemittel im Vakuum entfernt. Nach Filtration über 200 g Kieselgel (Hexan/Methylenchlorid) wurden 10 g (86%) 22 als farbloses Öl erhalten.
¹H-NMR (100 MHz, CDCl₃): 7,0 - 8,0 (m, 10H, arom. H), 6,6 (m, 1H, CH), 3,0 (m, 2H, CH₂), 2,0 (m, 3H, CH₃).

### 6. Dimethylbis(2-methyl-4-(1 -naphthyl)indenyl)silan (23)

Eine Lösung von 10 g (38 mmol) 22 in 100 cm³ H₂O- und O₂-freiem Toluol und 5 ml H₂O- und O₂-freiem THF wurden bei Raumtemperatur mit 14,4 cm³ (50 mmol) einer 20%igen Lösung von Butyllithium in Toluol versetzt und 2 h auf 80°C erhitzt. Anschließend wurde die gelbe Suspension auf 0°C gekühlt und mit 2,5 g (19 mmol) Dimethyldichlorsilan versetzt. Die Reaktionsmischung wurde noch 1 h auf 80°C erhitzt und anschließend mit 50 cm³ H₂O gewaschen. Das Lösemittel wurde im Vakuum entfernt und der Rückstand aus Heptan bei -20°C umkristallisiert. Es wurden 8,2 g (75%) 23 als farblose Kristalle erhalten.
¹H-NMR (100 MHz, CDCl₃): 7,2 - 8,1 (m, 20H, arom. H), 6,4 (m, 2H, H-C(3)), 4,0 (m, 2H, H-C(1)), -0,1, (s, 6H, CH₃Si).

### 7. rac-Dimethylsilandiylbis(2-methyl-4-(1-naphthyl)indenyl)zirkoniumdichlorid (24)

Eine Lösung von 8,0 g (14 mmol) 23 in 70 cm³ H₂O- und O₂-freiem Et₂O wurden unter Argon bei Raumtemperatur mit 10,5 cm³ einer 20%igen Lösung von Butyllithium in Toluol versetzt und anschließend 3 h zum Rückfluß erhitzt. Das Lösemittel wurde im Vakuum entfernt und der Rückstand mit 50 ml H₂O- und O₂-freiem Hexan über eine G3-Schlenkfritte filtriert, mit 50 ml H₂O- und O₂-freiem Hexan nachgewaschen und getrocknet (0,1 mbar, RT).

Das Dilithiosalz wurde bei -78°C zu einer Suspension von 3,2 g (14 mmol) Zirkoniumtetrachlorid in 80 cm³ Methylenchlorid gegeben und im Verlauf von 18 h unter magn. Rühren auf Raumtemperatur erwärmt. Der Ansatz wurde über eine G3-Fritte filtriert und der Rückstand portionsweise mit insgesamt 400 cm³ Methylenchlorid nachextrahiert. Die vereinigten Filtrate wurden im Vakuum vom Lösemittel befreit und aus Methylenchlorid umkristallisiert. Es wurden 1,5 g (15%) der racemischen und der meso-Form im Verhältnis 1:1 erhalten. Durch erneutes Umkristallisieren aus Methylenchlorid wurde der racemische Komplex in Form gelber Kristalle erhalten.
¹H-NMR (100 MHz, CDCl₃): 7,0 - 8,0 (m, 22 H, arom. H), 6,5 (s, 2H, H-C(3)), 2,2 (s, 6H, CH₃), 1,3 (s, 6H, CH₃Si). Massenspektrum: 729 M⁺, korrektes Zerfallsmuster.

### Beispiel F

### rac-Dimethylsilandiylbis(2-methyl-4-(2-naphthyl)indenyl)zirkoniumdichlorid (31)

### 1. 2-(2-Naphthyl)-toluol (25)

14 g (0,57 mol) Magnesium-Späne wurden mit 150 ml H₂O-freiem Et₂O überschichtet und die Grignard-Reaktion mit 5 g 2-Bromtoluol und einigen Körnchen Jod zum Anspringen gebracht. Anschließend wurden 95 g (0,58 mol) Bromtoluol in 450 ml H₂O-freiem Et₂O so zugetropft, daß die Reaktionsmischung am Sieden gehalten wurde. Nach vollständiger Zugabe wurde noch solange zum Sieden erhitzt, bis das Magnesium vollständig umgesetzt war.

Die Grignard-Lösung wurde anschließend zu einer Lösung von 120 g (0,57 mol) 2-Bromnaphthalin und 3,5 g Bis(triphenylphosphin)nickeldichlorid in 800 cm³ Toluol getropft, so daß die Innentemp 50°C nicht überstieg. Anschließend wurde noch 3 h zum Rückfluß erhitzt, mit 500 ml 10%iger wässr. HCI versetzt, die Phasen getrennt und die organische Phase im Vakuum vom Lösemittel befreit. Nach Filtration über Kieselgel (Hexan) wurden 107 g (87%) 25 als farbloses Öl erhalten.
¹H-NMR (100 MHz, CDCl₃): 7,0 - 7,9 (m, 11H, arom. H), 1,9 (s, 3H, CH₃).

### 2. 2-(2-Naphthyl)-benzylbromid (26)

105 g (0,48 mol) 25 und 90 g (0,5 mol) N-Bromsuccinimid wurden bei Raumtemperatur in 2000 cm³ Tetrachlorkohlenstoff gelöst, mit 3 g Azobisisobutyronitril versetzt und 4 h zum Rückfluß erhitzt. Das ausgefallene Succinimid wurde abfiltriert, das Lösemittel im Vakuum entfernt und der Rückstand durch Filtration über 1000 g Kieselgel (Hexan/Methylenchlorid 9:1) gereinigt. Es wurden 112 g (79%) 26 als farbloses, tränenreizendes Öl erhalten.
¹H-NMR (100 MHz, CDCl₃): 6,9 - 8,0 (m, 11H, arom. H), 4,1 (s, 2H, CH₂Br).

### 3. (±)-2-(2-(2-naphthyl)benzyl)-propionsäure (27)

Zu 8,5 g (0,37 mmol) Natrium in 100 cm³ H₂O-freiem EtOH wurden bei Raumtemperatur 70 g (0,37 mmol) Methyl-malonsäurediethylester gelöst in 50 cm³ H₂O-freiem EtOH zugetropft. Anschließend wurden 110 g (0,37 mmol) 26 in 200 cm³ H₂O-freiem EtOH zugetropft und 3 h zum Rückfluß erhitzt. Bei Raumtemperatur wurden 62 g (1,1 mol) KOH gelöst in 100 cm³ H₂O zugesetzt und weitere 4 h zum Rückfluß erhitzt. Die Lösemittel wurden im Vakuum entfernt, der Rückstand bis zur vollständigen Lösung mit H₂O versetzt und mit konzentrierter wässr. HCl bis pH 1 angesäuert. Der ausgefallene Niederschlag wurde abgesaugt, getrocknet und 1 h auf 130°C erhitzt. Man erhielt 90 g (84%) 27 als zähflüssiges Öl.
¹H-NMR (100 MHz, CDCl₃): 10,9 (s, 1H, COOH), 7,0-8,1 (m, 11H, arom. H), 2,3-3,0 (m, 3H, CH₂ und CH), 1,0 (d, 3H, CH₃).

### 4. (±)-2-Methyl-4-(2-naphthyl)-1-indanon (28)

Eine Lösung von 89 g (0,31 mol) 27 in 37 cm³ (0,5 mol) Thionylchlorid wurde 18 h bei Raumtemperatur gerührt. Überschüssiges Thionylchlorid wurde bei 10 mbar entfernt und der ölige Rückstand durch mehrmaliges Lösen in je 100 cm³ Toluol und Abziehen im Vakuum von anhaftenden Resten Thionylchlorid befreit.

Das Säurechlorid wurde in 200 cm³ Toluol aufgenommen und bei 10°C zu einer Suspension von 44 g (0,33 mol) AlCl₃ in 1000 cm³ Toluol getropft und 3 h auf 80°C erhitzt. Die Reaktionsmischung wurde auf 1000 g Eis gegossen und mit konz. wässr. HCl bis pH 1 angesäuert. Die organische Phase wurde abgetrennt und die wässr. Phase 3mal mit je 200 cm³ Methylenchlorid nachextrahiert. Die vereinigten organischen Phasen wurden mit gesättigter wässr. NaHCO₃-, gesättigter wässr. NaCl-Lösung gewaschen und anschließend getrocknet (MgSO₄). Nach Chromatographie an 1000 g Kieselgel (Hexan/AeOEt) wurden 27 g (33%) 28 erhalten.
¹H-NMR (100 MHz, CDCl₃): 7,1 - 8,0 (m, 10H, arom. H), 2,2 - 3,3 (m, 3H, CH₂ und CH), 1,1 (d, 3H, CH₃).

### 5. 2-Methyl-7-(2-naphthyl)inden (29)

Zu einer Lösung von 27 g (100 mmol) 28 in 200 cm³ THF/Methanol 2:1 wurden bei 0°C 3,8 g (100 mmol) NaBH₄ zugesetzt und 18 h bei Raumtemperatur gerührt. Die Reaktionsmischung wurde auf 100 g Eis gegossen, mit konz. wässr. HCl bis pH 1 versetzt und mehrmals mit Et₂O extrahiert. Die vereinigten organischen Phasen wurden mit gesättigter wässr. NaHCO₃-, gesättigter wässr. NaCl-Lösung gewaschen und anschließend getrocknet (MgSO₄).

Das Rohprodukt wurde in 500 cm³ Toluol aufgenommen, mit 1,5 g p-Toluolsulfonsäure versetzt und 2 h am Wasserabscheider zum Rückfluß erhitzt. Die Reaktionsmischung wurde 3mal mit 50 cm³ gesättigter wässr. NaHCO₃-Lösung gewaschen und das Lösemittel im Vakuum entfernt. Nach Filtration über 200 g Kieselgel (Hexan/Methylenchlorid) wurden 18,4 g (72%) 29 als farbloses Öl erhalten.
¹H-NMR (100 MHz, CDCl₃): 7,0 - 8,0 (m, 10H, arom. H), 6,6 (m, 1H, CH), 3,0 (m, 2H, CH₂), 2,0 (m, 3H, CH₃).

### 6. Dimethylbis(2-methyl-4-(2-naphthyl)indenyl)silan (30)

Eine Lösung von 18 g (70 mmol) 29 in 70 cm³ H₂O- und O₂-freiem Toluol und 4 ml H₂O- und O₂-freiem THF wurden bei Raumtemperatur mit 26 cm³ (70 mmol) einer 20%igen Lösung von Butyllithium in Toluol versetzt und 2 h auf 80°C erhitzt. Anschließend wurde die gelbe Suspension auf 0°C gekühlt und mit 4,5 g (35 mmol) Dimethyldichlorsilan versetzt. Die Reaktionsmischung wurde noch 1 h auf 80°C erhitzt und anschließend mit 50 cm³ H₂O gewaschen. Das Lösemittel wurde im Vakuum entfernt und der Rückstand aus Heptan bei -20°C umkristallisiert. Es wurden 10,8 g (54%) 30 als farblose Kristalle erhalten.
¹H-NMR (100 MHz, CDCl₃): 7,0 - 8,1 (m, 20H, arom. H), 6,4 (m, 2H, H-C(3)), 4,0 (m, 2H, H-C(1)), -0,1, (s, 6H, CH₃Si).

### 7. rac-Dimethylsilandiylbis(2-methyl-4-(2-naphthyl)indenyl)zirkoniumdichlorid (31)

Eine Lösung von 10,5 g (18 mmol) 30 in 70 cm³ H₂O- und O₂-freiem Et₂O wurden unter Argon bei Raumtemperatur mit 13,6 cm³ einer 20%igen Lösung von Butyllithium in Toluol versetzt und anschließend 3 h zum Rückfluß erhitzt. Das Lösemittel wurde im Vakuum entfernt und der Rückstand mit 50 ml H₂O- und O₂-freiem Hexan über eine G3-Schlenkfritte filtriert, mit 50 ml H₂O- und O₂-freiem Hexan nachgewaschen und getrocknet (0,1 mbar, RT).

Das Dilithiosalz wurde bei -78°C zu einer Suspension von 4,2 g (18 mmol) Zirkoniumtetrachlorid in 80 cm³ Methylenchlorid gegeben und im Verlauf von 18 h unter magn. Rühren auf Raumtemperatur erwärmt. Der Ansatz wurde über eine G3-Fritte filtriert und der Rückstand portionsweise mit insgesamt 400 cm³ Methylenchlorid nachextrahiert. Die vereinigten Filtrate wurden im Vakuum vom Lösemittel befreit und aus Methylenchlorid umkristallisiert. Es wurden 3,1 g (23%) der racemischen und der meso-Form im Verhältnis 1:1 erhalten. Durch erneutes Umkristallisieren aus Methylenchlorid wurde der racemische Komplex in Form gelber Kristalle erhalten.
¹H-NMR (100 MHz, CDCl₃): 7,0 - 8,0 (m, 22 H, arom. H), 6,9 (s, 2H, H-C(3)), 2,2 (s, 6H, CH₃), 1,3 (s, 6H, CH₃Si). Massenspektrum: 729 M⁺, korrektes Zerfallsmuster.

### Beispiel G

### rac-Ethandiylbis(2-methyl-4-phenylindenyl)zirkoniumdichlorid (33)

### 1. 1,2-Bis(2-methyl-4-phenylindenyl)ethan (32)

Eine Lösung von 50 g (0,24 mol) 3 in 500 ml THF wurde unter Argon bei Raumtemperatur mit 90 cm³ (0,24 mol) einer 20%igen Lösung von Butyllithium in Toluol versetzt und 2 h bei 60°C nachgerührt. Es wurde auf -78°C abgekühlt, 22,5 g (0,12 mol) 1,2-Dibromethan zugesetzt und im Verlauf von 18 h auf Raumtemperatur erwärmt. Die Reaktionsmischung wurde mit 50 cm³ H₂O gewaschen, das Lösemittel im Vakuum entfernt und der Rückstand an 500 g Kieselgel (Hexan/Methylenchlorid 9:1) chromatographiert. Es wurden 2,5 g (5%) 32 als gelbes Öl erhalten, das bei -20°C langsam erstarrte.
¹H-NMR (100 MHz, CDCl₃): 7,0 - 8,1 (m, 20H, arom. H), 6,4 (m, 2H, H-C(3)), 4,0 (m, 2H, H-C(1)), -0,1, (s, 6H, CH₃Si).

### 2. rac-Ethandiylbis(2-methyl-4-phenylindenyl)zirkoniumdichlorid (33)

Eine Lösung von 2,3 g (5 mmol) 32 in 20 ml H₂O- und O₂-freiem Et₂O wurde unter Argon bei Raumtemperatur mit 4 cm³ (10 mmol) einer 20%igen Lösung von Butyllithium in Toluol versetzt und 3 h zum Rückfluß erhitzt. Das Lösemittel wurde im Vakuum entfernt und der Rückstand mit 30 ml H₂O- und O₂-freiem Hexan über eine G3-Schlenkfritte filtriert, mit 30 ml H₂O- und O₂-freiem Hexan nachgewaschen und getrocknet (0,1 mbar, RT).

Das Dilithiosalz wurde bei -78°C zu einer Suspension von 1,2 g (5 mmol) Zirkoniumtetrachlorid in 30 cm³ Methylenchlorid gegeben und im Verlauf von 18 h unter magn. Rühren auf Raumtemperatur erwärmt. Der Ansatz wurde über eine G3-Fritte filtriert und der Rückstand portionsweise mit insgesamt 100 cm³ Methylenchlorid nachextrahiert. Die vereinigten Filtrate wurden im Vakuum vom Lösemittel befreit und aus Methylenchlorid/Hexan umkristallisiert. Es wurden 0,5 g (18%) der racemischen und der meso-Form im Verhältnis 1:1 erhalten. Durch erneutes Umkristallisieren aus Toluol wurde der racemische Komplex in Form gelber Kristalle erhalten.
¹H-NMR (100 MHz, CDCl₃): 7,0 - 7,7 (m, 16H, arom. H), 6,6 (m, 2H, H-C(3)), 3,4-4,1 (m, 4H, H₂C-CH₂), 2,1 (s, 6H, CH₃). Massenspektrum: 598 M⁺, korrektes Zerfallsmuster.

### Beispiel H

### Me₂Si(2-Me-4-Ph-Indenyl)₂ZrMe[BPh₄] (35)

### 1. rac-Dimethylsilandiylbis(2-Methyl-4-phenyl-indenyl)zirkoniumdimethyl (34)

0,5 g (0,8 mmol) rac-5 wurden in 10 cm³ H₂O- und O₂-freiem Et₂O bei -30°C mit 1 cm³ einer 1,6 M (1,6 mmol) Lösung von Methyllithium in Et₂O versetzt und 1 h bei 0°C gerührt. Anschließend wurde das Lösemittel im Vakuum entfernt, der Rückstand in 20 cm³ H₂O- und O₂-freiem Hexan aufgenommen und über eine G3-Fritte abfiltriert. Es wurden 0,34 g (72%) 34 erhalten. Massenspektrum: 588 M⁺, korrektes Zerfallsmuster.

### 2. Me₂Si(2-Me-4-Ph-lndenyl)₂ZrMe[BPh₄] (35)

0,2 g (0,3 mmol) 34 wurden bei 0°C zu 0,25 g (mmol) Tributylammoniumtetraphenylborat in 30 cm³ Toluol gegeben. Unter Rühren wurde auf 50°C erwärmt und die Mischung 15 Minuten bei dieser Temperatur gerührt. Für die Polymerisation wurde ein aliquoter Teil der Lösung verwendet.

### Beispiel 1

Ein trockener 16-dm³-Reaktor wurde zunächst mit Stickstoff und anschließend mit Propylen gespült und mit 10 dm³ flüssigem Propylen befüllt. Dann wurden 30 cm³ toluolische Methylaluminoxanlösung zugegeben und der Ansatz bei 30°C 15 Minuten gerührt.

Parallel dazu wurden 1,1 mg rac-5 in 20 cm³ toluolischer Methylaluminoxanlösung (27 mmol Al) gelöst und durch 15 minütiges Stehenlassen zur Reaktion gebracht. Die Lösung wurde dann in den Reaktor gegeben, durch Wärmezufuhr auf die Polymerisationstemperatur von 50°C aufgeheizt (4°C/min) und das Polymerisationssystem 1 h durch Kühlung bei 50°C gehalten. Gestoppt wurde die Polymerisation durch Zusatz von 20 cm³ Isopropanol. Das überschüssige Monomer wurde abgegast, das Polymer im Vakuum getrocknet. Man erhielt 0,9 kg Polypropylen. Der Reaktor zeigte dünne Beläge an Innenwand und Rührer. Die Katalysatoraktivität betrug 818 kg PP/g Metallocen x h. VZ = 905 cm³/g; Schmp. = 159,4°C; II = 98,8%; mmmm = 95,4%; M_{w} = 1100000 g/mol; M_{w}/Mₙ = 2,5.

### Beispiel 2

Die Polymerisation aus Beispiel 1 wurde wiederholt mit dem Unterschied, daß als Katalysator 0,9 mg rac-5 verwendet wurde und die Polymerisationstemperatur 70 °C betrug. Man erhielt 1,4 kg Polypropylen. Der Reaktor zeigte starke Beläge an Innenwand und Rührer. Die Katalysatoraktivität betrug 1555 kg PP/g Metallocen x h. VZ = 719 cm³/g; Schmp. = 157,7°C.

### Beispiel 3

22 cm³ der Suspension des "MAO auf SiO₂" (49 mmol Al) wurde unter Argon in eine G3-Schlenkfritte eingefüllt und mit einer Lösung von 4,5 mg rac-5 in 10 cm³ Toluol (7,2 µmol Zr) versetzt.
Das Reaktionsgemisch wurde 30 Minuten bei Raumtemperatur gerührt, wobei eine spontane Farbänderung nach rot allmählich verblaßte. Anschließend wurde das Gemisch filtriert und der Feststoff 3 mal mit 10 cm³ Hexan gewaschen. Der verbleibende, hexanfeuchte Filterrückstand wurde für die Polymerisation erneut in 20 cm³ Hexan resuspendiert.

Parallel dazu wurde ein trockener 16-dm³-Reaktor zunächst mit Stickstoff und anschließend mit Propylen gespült und mit 10 dm³ flüssigem Propylen befüllt.
Dann wurden 3 cm³ Triisobutylaluminium (pur, 12 mmol) mit 30 cm³ Hexan verdünnt, in den Reaktor gegeben und der Ansatz bei 30°C 15 Minuten gerührt.
Anschließend wurde die Katalysator-Suspension in den Reaktor gegeben, auf die Polymerisationstemperatur von 50°C aufgeheizt (4°C/min) und das Polymerisationssystem 1 h durch Kühlung bei 50°C gehalten. Gestoppt wurde die Polymerisation durch Zusatz von 20 cm³ Isopropanol. Das überschüssige Monomer wurde abgegast, das Polymer im Vakuum getrocknet.
Es resultierten 300 g Polypropylen- Pulver. Der Reaktor zeigte keine Beläge an Innenwand oder Rührer. Die Katalysatoraktivität betrug 67 kg PP/g Metallocen x h. VZ = 1380 cm³/g; Schmp. = 156°C.

### Beispiel 4

Die Synthese des geträgerten Katalysatorsystems aus Beispiel 3 wurde wiederholt mit dem Unterschied, daß 13 cm³ (29 mmol Al) der Suspension "MAO auf SiO₂" und 1,8 mg rac-5 (2,9 µmol Zr) verwendet wurden.

Die Polymerisation erfolgte analog zu Beispiel 3 bei 70°C. Es resultierten 420 g Polypropylen-Pulver. Der Reaktor zeigte keine Beläge an Innenwand oder Rührer. Die Katalysatoraktivität betrug 233 kg PP/g Metallocen x h. VZ = 787 cm³/g; Schmp. = 149,5°C.

### Beispiel 5

Die Synthese des geträgerten Katalysatorsystems aus Beispiel 3 wurde wiederholt mit dem Unterschied, daß 150 cm³ (335 mmol Al) der Suspension "MAO auf SiO₂" und 44,2 mg rac-5 (70,3 µmol Zr) verwendet und das Reaktionsgemisch 60 Minuten bei Raumtemperatur gerührt wurde. Anschließend wurde der Feststoff abfiltriert und 3 mal mit 50 cm³ Hexan gewaschen. Der verbleibende, hexanfeuchte Filterrückstand wurde am Vakuum zu einem frei fließenden, blaßrosa Pulver getrocknet. Man erhielt 33,3 g geträgerten, trockenen Katalysator.

Für die Polymerisation wurden von diesem trockenen Katalysator 2,98 g (4 mg = 6,3 µmol Zr) erneut in 20 cm³ Hexan resuspendiert.

Die Polymerisation erfolgte analog zu Beispiel 3 bei 70°C.
Es resultierten 1,05 kg Polypropylen-Pulver. Der Reaktor zeigte keine Beläge an Innenwand oder Rührer. Die Katalysatoraktivität betrug 263 kg PP/g Metallocen x h. Vz = 944 cm³/g; Schmp. = 156°C.

### Beispiel 6

Ein trockener 1,5 dm³ - Reaktor wurde mit N₂ gespült und bei 20°C mit 750 cm³ eines entaromatisierten Benzinschnittes mit dem Siedebereich 100 -120°C ("®Exxsol 100/120") gefüllt. Dann wurde der Gasraum des Reaktors durch 5 - maliges Aufdrücken von 8 bar Propylen und Entspannen stickstofffrei gespült. Danach wurden 3,75 cm³ toluolische Methylaluminoxanlösung (10 Gew% MAO) zugegeben. Unter Rühren wurde der Reaktorinhalt innerhalb von 15 min auf 30°C aufgeheizt und durch Zugabe von Propylen bei einer Rührgeschwindigkeit von 500 UPM der Gesamtdruck auf 8 bar eingestellt.

Parallel dazu wurden 0,1 mg rac-5 in 1,25 cm³ toluolischer Methylaluminoxanlösung gelöst und durch 15 minütiges Stehenlassen zur vollständigen Reaktion gebracht. Dann wurde die Lösung in den Reaktor gegeben, das Polymerisationssystem auf eine Temperatur von 50°C gebracht und durch entsprechende Kühlung 1 h bei dieser Temperatur gehalten. Durch entsprechende Zufuhr von Propylen wurde der Druck während dieser Zeit bei 8 bar gehalten, danach die Reaktion durch Zugabe von 2 cm³ Isopropanol gestoppt, das Polymere abfiltriert und im Vakuum getrocknet.

Man erhielt 16 g Polypropylen. Der Reaktor zeigte Beläge an Innenwand und Rührer. Die Katalysatoraktivität (KZA_{red}) betrug 20 kg PP/g Metallocen x h x bar. VZ = 833 cm³/g; Schmp. = 159°C.

### Beispiel 7

Die Polymerisation aus Beispiel 6 wurde wiederholt mit dem Unterschied, daß die Polymerisationstemperatur 60°C betrug.

Man erhielt 35 g Polypropylen. Der Reaktor zeigte Beläge an Innenwand und Rührer. Die Katalysatoraktivität (KZA_{red}) betrug 44 kg PP/g Metallocen x h x bar. VZ = 484 cm³/g; Schmp. = 159°C.

### Beispiel 8

Die Polymerisation aus Beispiel 6 wurde wiederholt mit dem Unterschied, daß die Polymerisationstemperatur 70°C betrug.

Man erhielt 88 g Polypropylen. Der Reaktor zeigte Beläge an Innenwand und Rührer. Die Katalysatoraktivität (KZA_{red}) betrug 110 kg PP/g Metallocen x h x bar. VZ = 414 cm³/g; Schmp. = 159°C.

### Beispiele 9-12

Es wurde verfahren wie in Beispiel 2. Vor der Befüllung mit flüssigem Propylen wurde jedoch Wasserstoff zudosiert:

| Beispiel | Ndm²H₂ | Metallocenaktivität [kgPP/gMet*h] | VZ [cm³/g] |
|---|---|---|---|
| 9 | 1,5 | 1640 | 495 |
| 10 | 3 | 1590 | 212 |
| 11 | 4,5 | 1720 | 142 |
| 12 | 200 | 1580 | 17 |

Die Beispiele 9-12 zeigen die gute Wasserstoffansprechbarkeit des erfingungsgemäßen Metallocens. Es ist eine Molmassenregelung bis in den Wachsbereich (s. Beispiel 12) möglich.

### Beispiel 13

Es wurde verfahren wie in Beispiel 3. Vor der Zugabe des Katalysators wurde jedoch 0,2 bar Wasserstoff auf den Reaktor gedrückt, die Polymerisationstemperatur betrug 60°C. Während der Polymerisation wurde jedoch gleichmäßig Ethylen zudosiert. Insgesamt wurden 12 g Ethylen in den Reaktor gegeben. Es wurden 0,4 kg Ethylen-Propylen-Copolymer erhalten. Die Metallocenaktivität war 88 kg Copolymer/g Metallocen x h. Der Ethylengehalt im Polymeren betrug 2,4 Gew.-%, das Ethylen wurde überwiegend isoliert eingebaut. VZ = 200 cm³/g; Schmelzpunkt 143°C.

### Beispiel 14

Es wurde verfahren wie in Beispiel 13. Während der Polymerisation wurden jedoch insgesamt 34 g Ethylen zudosiert. Es wurden 0,38 kg Ethylen-Propylen-Copolymer mit 7 Gew.-% Ethylen erhalten. VZ = 120 cm³/g; Schmelzpunkt 121°C.

### Beispiel 15

Es wurde verfahren wie in Beispiel 4. Während der Polymerisation wurden jedoch 4 g Ethylen zudosiert und vor der Polymerisation 0,1 bar Wasserstoff aufgedrückt. Es wurden 0,52 kg Ethylen-Propylen-Copolymer erhalten. Die Metallocenaktivität war 286 kg Copolymer/g Metallocen x h. Der Ethylengehalt im Polymeren betrug 6,1 Gew.-%, das Ethylen wurde zu einem größeren Teil isoliert eingebaut. VZ = 150 cm³/g; Schmelzpunkt 116°C.

### Beispiel 16

Ein trockener 150 dm³ Reaktor wurde mit Stickstoff gespült und bei 20°C mit 80 dm³ eines entaromatisierten Benzinschnittes mit dem Siedebereich 100-120°C gefüllt.

Dann wurde der Gasraum durch 5maliges Aufdrücken von 2 bar Propylen und Entspannen Stickstoff-frei gespült. Nach Zugabe von 50 l flüssigem Propylen wurden 64 cm³ toluolische Methylaluminoxanlösung (entsprechend 100 mmol Al, Molmasse nach kryoskopischer Bestimmung 1080 g/mol) zugegeben und der Reaktorinhalt auf 50°C aufgeheizt. Durch Zudosierung von Wasserstoff wurde ein Wasserstoffgehalt im Gasraum des Reaktors von 2,0% eingestellt und später dann während der 1. Polymerisationsstufe durch Nachdosierung konstant gehalten.

9,8 mg rac-7 wurden in 32 ml der toluolischen Methylaluminoxanlösung (entsprechend 50 mmol Al) gelöst und nach 15 Minuten in den Reaktor gegeben. Die Polymerisation erfolgte nun in einer 1. Polymerisationsstufe bei 50°C 5 h lang. Danach wurde auf 3 bar Reaktordruck abgegast und 2000 g Ethylengas zugeführt. Der Reaktordruck stieg dabei auf 8 bar an und bei 40°C wurden weitere 14 h polymerisiert, bevor die Reaktion mit CO₂-Gas gestoppt wurde.
Es wurden 18,6 kg Blockcopolymer erhalten, entsprechend einer Metallocenaktivität von 99,9 kg Copolymer/g Metallocen x h. VZ=230cm³/g; MFI (230/5) = 11dg/min, MFI (230/2.16) = 3,7 dg/min; Schmelzpunkt des Polymers der 1. Polym.-Stufe: 159°C, Glastemperatur des Polymers der 2. Polym.-Stufe: -38°C. Das Blockcopolymer enthielt 5% Ethylen. Die Fraktionierung des Produktes ergab folgende Zusammensetzung: 69 Gew.-% Homopolymer, 31 Gew.-% Copolymer, wobei das Copolymer einen Ethylengehalt von 15 Gew.-% aufwies, die mittlere Blocklänge C₂ war 2,2.

### Beispiel 16 a

### Es wurde verfahren wie in Beispiel 16.

3 mg rac-24 wurden in 32 ml der toluolischen Methylaluminoxanlösung (entsprechend 50 mmol Al) gelöst und nach 15 Minuten in den Reaktor gegeben. Die Polymerisation erfolgte nun in einer 1. Polymerisationsstufe bei 50°C 2,5 h lang. Danach wurde auf 3 bar Reaktordruck abgegast und 3000 g Ethylengas zugeführt. Der Reaktordruck stieg dabei auf 8 bar an und bei 40°C wurden weitere 8 h polymerisiert, bevor die Reaktion mit CO₂-Gas gestoppt wurde.

Es wurden 16,5 kg Blockcopolymer erhalten, entsprechend einer Metallocenaktivität von 524 kg Copolymer/g Metallocen x h. VZ = 480 cm³/g; MFI (230/5) = 2 dg/min, Schmelzpunkt des Polymers der 1. Polym.-Stufe: 162°C, Glastemperatur des Polymers der 2. Polym.-Stufe: -54°C. Das Blockpolymer enthielt 15% Ethylen.

### Beispiel 17

Es wurde verfahren wie in Beispiel 1. Verwendet wurden jedoch 12,5 mg Metallocen rac-7. Man erhielt 1,5 kg Polypropylen, die Metallocenaktivität war 120 kg PP/g Metallocen x h. VZ = 1050 cm³/g; Schmelzpunkt 159°C.

### Beispiel 18

Es wurde verfahren wie in Beispiel 2. Verwendet wurden jedoch 4,1 mg Metallocen rac-7. Man erhielt 1,3 kg Polypropylen, die Metallocenaktivität war 317 kg PP/g Metallocen x h. VZ = 555 cm³/g; Schmelzpunkt 157°C.

### Vergleichsbeispiel A

Es wurde verfahren wie in Beispiel 1. Verwendet wurden jedoch 12,5 mg rac-Phenyl(methyl)silandiylbis(2-methyl-1-indenyl)zirkoniumdichlorid. Man erhielt 1,35 kg Polypropylen, die Metallocenaktivität war 108 kg PP/g Metallocen x h. VZ = 1050 cm³/g; Schmelzpunkt 149°C.

### Vergleichsbeispiel B

Es wurde verfahren wie in Beispiel 1. Verwendet wurden jedoch 12,5 mg rac-Phenyl (methyl)silandiylbis(1-indenyl)zirkoniumdichlorid. Man erhielt 0,28 kg Polypropylen, die Metallocenaktivität war 22,4 kg PP/g Metallocen x h. VZ = 74 cm³/g; Schmelzpunkt 141°C.

### Beispiel 19

Es wurde verfahren wie in Beispiel 1. Verwendet wurden jedoch 3,3 mg 24. Man erhielt 0,78 kg Polypropylen, die Metallocenaktivität war 237 kg PP/g Metallocen x h. VZ = 1700 cm³/g; Schmelzpunkt 163°C, M_{w} = 2,1*10⁶ g/mol, MFI 230/21.6 = 1 dg/min; M_{w}/Mₙ = 2,1.

### Beispiel 19 a

Es wurde verfahren wie in Beispiel 2. Verwendet wurden jedoch 1.0 mg rac-24. Man erhielt 1.2 kg Polypropylen. Die Metallocenaktivität war 1200 kg PP/g Metallocen x h. VZ = 1100 cm³/g. Schmelzpunkt = 161°C.

### Beispiel 20

Es wurde verfahren wie in Beispiel 1; die Polymerisationstemperatur war jedoch 40°C. Verwendet wurden 6,0 mg 17. Man erhielt 1,95 kg Polypropylen, die Metallocenaktivität war 325 kg PP/g Metallocen x h. VZ = 1320 cm³/g; Schmelzpunkt 162°C, M_{w} = 1,79∗10⁶ g/mol, M_{w}/Mₙ = 2,3.

### Vergleichbeispiel C

Es wurde verfahren wie in Beispiel 20. Verwendet wurde jedoch das nicht erfindungsgemäße Metallocen rac-Dimethylsilandiylbis(2-ethyl-1-indenyl)zirkoniumdichlorid. Man erhielt 0,374 kg Polypropylen, die Metallocenaktivität war 62,3 kg PP/g Metallocen x h. VZ = 398 cm³/g; Schmelzpunkt 147°C, M_{w} = 450.000 g/mol, M_{w}/Mₙ = 2,5.

### Beispiel 21

Es wurde verfahren wie in Beispiel 1. Verwendet wurden jedoch 5,2 mg 31. Man erhielt 1,67 kg Polypropylen, die Metallocenaktivität war 321 kg PP/g Metallocen x h. VZ = 980 cm³/g; Schmelzpunkt 158°C.

### Beispiel 22

Es wurde verfahren wie in Beispiel 1, Die Polymerisation wurde jedoch bei 30°C durchgeführt. Verwendet wurden jedoch 3,7 mg 33. Man erhielt 0,35 kg Polypropylen, die Metallocenaktivität war 94 kg PP/g Metallocen x h. VZ = 440 cm³/g; Schmelzpunkt 153°C.

### Beispiel 23

Ein trockener 16 dm³ Reaktor wurde mit Propylen gespült und mit 10 dm³ flüssigem Propylen befüllt. Dann wurden 1,1 cm³ des Reaktionsprodukts aus H.2 (entsprechend 7,5 mg 34) in 20 cm³ Toluol gelöst und bei 30°C in den Reaktor gegeben. Der Reaktor wurde auf 50°C aufgeheizt (10°C/min) und das Polymerisationssystem 1h durch Kühlung bei dieser Temperatur gehalten. Gestoppt wurde die Polymerisation durch Zugabe von CO₂-Gas. Das überschüssige Monomere wurde abgegast und das Polymer im Vakuum bei 80°C getrocknet. Man erhielt 2,45 kg Polypropylen. VZ = 875 cm³/g; Schmelzpunkt 160°C.

### Beispiel 24

Ein trockener 16 dm³-Reaktor wurde mit Stickstoff gespült und bei 20°C mit 10 dm³ eines entaromatisierten Benzinschnittes mit dem Siedebereich 100-120°C gefüllt. Dann wurde der Gasraum des Reaktors durch 5maliges Aufdrücken von 2 bar Ethylen und Entspannen stickstofffrei gespült. Dann wurden 30 cm³ toluolische Methylaluminoxanlösung (entsprechend 45 mmol Al, Molmasse nach kryoskopischer Bestimmung 700 g/mol) zugegeben. Unter Rühren wurde der Reaktorinhalt innerhalb von 15 Minuten auf 30°C aufgeheizt und durch Zugabe von Ethylen wurde bei 250 Upm Rührgeschwindigkeit der Gesamtdruck auf 5 bar eingestellt.

Parallel dazu wurden 3,2 g 12 in 20 cm³ toluolischer Methylaluminoxanlösung gelöst und durch 15 minütiges Stehenlassen voraktiviert. Dann wurde die Lösung in den Reaktor gegeben, das Polymerisationssystem wurde auf eine Temperatur von 50°C gebracht und durch entsprechende Kühlung 4 h bei dieser Temperatur gehalten. Der Gesamtdruck wurde während dieser Zeit durch entsprechende Zufuhr von Ethylen bei 5 bar gehalten.

Die Polymerisation wurde durch Zugabe von 20 ml Isopropanol gestoppt, das Polymere abfiltriert und im Vakuum getrocknet. Man erhielt 0,7 kg Polyethylen. VZ = 690 cm³/g.

### Beispiel 25

Es wurde die Vorschrift von Beispiel 24 befolgt. In Abweichung von Beispiel 23 wurde 1,8 mg rac-7 eingesetzt, das Polymerisationssystem auf 70°C gebracht und 1 h bei dieser Temperatur gehalten. Man erhielt 0,9 kg Polyethylen. VZ = 730 cm³/g.

### Beispiel 26

15g "F-MAO auf SiO2" (111 mmol Al) wurden in einem rührbaren Gefäß in 100 cm³ Toluol suspendiert und auf -20°C abgekühlt. Gleichzeitig wurden 155mg (0,246 mmol) rac-5 in 75 cm³ Toluol gelöst und innerhalb von 30 Minuten zur Suspension zugetropft. Es wurde langsam unter Rühren auf Raumtemperatur erwärmt, wobei die Suspension eine rote Farbe annahm. Anschließend wurde eine Stunde bei 80°C gerührt und nach dem Abkühlen auf Raumtemperatur wurde das Gemisch filtriert und der Feststoff 3mal mit je 100cm³ Toluol und 1 mal mit 100cm³ Hexan gewaschen. Das Filtrat war rot. Der verbleibende, hexanfeuchte Filterrückstand wurde im Vakuum getrocknet. Man erhielt 13,2 g frei fließenden, hellroten, geträgerten Katalysator. Die Analyse ergab einem Gehalt von 3,2 mg Zirkonocen pro Gramm Katalysator.

Polymerisation: Für die Polymerisation wurden 2,08 g des Katalysators in 50 cm³ eines entaromatisierten Benzinschnittes mit dem Siedebereich 100 -120°C suspendiert. Die Polymerisation erfolgte analog zu Beispiel 3 bei 60°C. Es wurden 1100g Polypropylen-Pulver erhalten. Der Reaktor zeigte keine Beläge an Innenwand oder Rührer. Aktivität = 165 kg PP/(g Metallocen x h). VZ = 1100 cm³/g. Schmelzpunkt = 153°C; M_{w} = 1.485.000; M_{w}/Mₙ = 3,2; MFI 230/5 = 0,1 dg/min; SD = 440 g/dm³.

### Beispiel 27

1,31g des Katalysators aus Beispiel 26 wurden in 50 cm³ eines entaromatisierten Benzinschnittes mit dem Siedebereich 100 -120°C suspendiert. Die Polymerisation erfolgte analog zu Beispiel 3 bei 70°C. Man erhielt 1300g Polypropylen-Pulver. Der Reaktor zeigte keine Beläge an Innenwand oder Rührer. Aktivität = 310 kg PP/(g Metallocen x h). VZ = 892 cm³/g; Schmelzpunkt = 150°C, M_{w} = 1.290.000; M_{w}/Mₙ = 3,0; SD = 410 g/dm³.

### Beispiel 28

Die Trägerung aus Beispiel 26 wurde wiederholt mit dem Unterschied, daß 0,845 g rac-5 gelöst in 500 cm³ Toluol mit 90g "F-MAO auf SiO₂" suspendiert in 500 cm³ Toluol zur Reaktion gebracht wurde. Man erhielt 84 g roten, pulvrigen Katalysator. Die Analyse ergab einen Gehalt von 9 mg Metallocen pro Gramm Feststoff, das rote Filtrat enthielt 13 mg Zirkonium.

Polymerisation: 1,1g des geträgerten Katalysators wurden in 50ml eines entaromatisierten Benzinschnittes mit dem Siedebereich 100 -120°C suspendiert. Die Polymerisation erfolgte analog zu Beispiel 3 bei 70°C. Man erhielt 2850 g Polypropylen-Pulver. Der Reaktor zeigte keine Beläge an Innenwand oder Rührer. Aktivität = 288 kg PP/(g Metallocen x h); VZ = 638 cm³/g; Schmelzpunkt = 150°C; MFI 230/5 = 0,5 dg/min; SD = 410 g/dm³.

### Beispiel 29

Ein mikroporöses Polypropylenpulver (AKZO) mit einer Teilchengröße kleiner 100 µm wurde durch Extraktion mit Toluol in einem Soxhlet-Extraktor unter Inertbedingungen von Verunreinigungen befreit und anschließend mit 20 Gew.%iger Trimethylaluminiumlösung in Toluol gewaschen und im Vakuum getrocknet. Parallel dazu wurden 51,lmg rac-5 in 40 cm³ toluolischer Methylaluminoxanlösung gelöst und durch 15 minütiges Stehenlassen zur vollständigen Reaktion gebracht. 16,5 g des PP-Pulvers wurden zudosiert und durch kurzzeitiges Anlegen eines Vakuums wurde das in den Poren des Trägers befindliche Gas und ein Teil des Lösungsmittels entfernt und die Katalysatorlösung vollständig aufgesogen. Durch intensives Schütteln des Reaktionsgefäßes erhielt man 46 g homogenes, feinteiliges und gut fließendes rotes Pulver. 10 g des geträgerten Katalysatorpulvers wurden unter Inertbedingungen in einem Rotationsverdampfer mit Ethylen 30 Minuten vorpolymerisiert. Der Ethylenüberdruck wurde durch ein Druckregelventil konstant bei 0,1 bar gehalten, die Durchmischung des Katalysatorpulvers erfolgte durch kontinuierliche Rotation des Reaktionsgefäßes unter Kühlung auf 0°C. Es resultierten 12 g vorpolymerisierter Katalysator.

Polymerisation: 4,6g des geträgerten, vorpolymerisierten Katalysators wurden in 50 cm³ eines entaromatisierten Benzinschnittes mit dem Siedebereich 100 -120°C suspendiert. Die Polymerisation erfolgte analog zu Beispiel 3 bei 70°C. Es resultierten 250 g Polypropylen-Pulver. Der Reaktor zeigte keine Beläge an Innenwand oder Rührer, der mittlere Teilchendurchmesser war 1000 µm. Aktivität = 59 kg PP/(g Metallocen x h); VZ = 734 cm³/g. Schmelzpunkt = 152°C; SD = 390 g/dm³.

### Beispiel 30

1 g des geträgerten, nicht vorpolymerisierten Katalysators aus Beispiel 29 wurden in 50 cm³ n-Decan für die Polymerisation suspendiert. Die Polymerisation erfolgte analog zu Beispiel 3 bei 70°C. Es resultierten 600 g Polypropylen. Der Reaktor zeigte dünne Beläge an Innenwand und Rührer, der mittlere Teilchendurchmesser war > 2000 µm. Aktivität = 540 kg PP/(g Metallocen x h); VZ = 1400 cm³/g;Schmelzpunkt = 157,7°C; SD = 280 g/dm³.

## Patentansprüche

1. Verbindung der Formel I 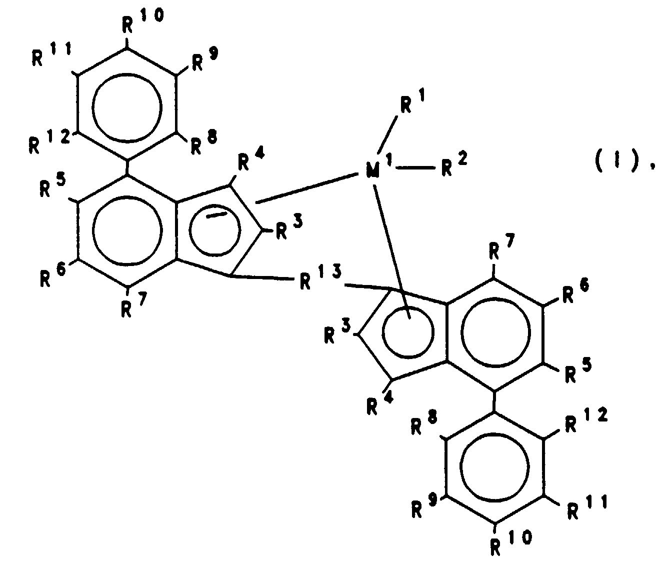 worin
M¹ ein Metall der Gruppe IVb, Vb oder Vlb des Periodensystems ist,
R¹ und R² gleich oder verschieden sind und ein Wasserstoffatom, eine C₁-C₁₀-Alkyl-, eine C₁-C₁₀-Alkoxy-, eine C₆-C₁₀-Aryl-, eine C₆-C₁₀-Aryloxy-, eine C₂-C₁₀-Alkenyl-, eine C₇-C₄₀-Arylalkyl-, eine C₇-C₄₀-Alkylaryl-, eine C₈-C₄₀-Arylalkenyl-, eine OH-Gruppe oder ein Halogenatom bedeuten,
die Reste R³ gleich oder verschieden sind und ein Wasserstoffatom, ein Halogenatom, eine C₁-C₁₀-Alkylgruppe, die halogeniert sein kann, eine C₆-C₁₀-Arylgruppe, einen NR¹⁶₂-, -SR¹⁶-, -OSiR¹⁶₃-, -SiR¹⁶₃- oder -PR¹⁶₂-Rest bedeuten, worin R¹⁶ ein Halogenatom, eine C₁-C₁₀-Alkylgruppe oder eine C₆-C₁₀-Arylgruppe ist,
R⁴ bis R¹² gleich oder verschieden sind und die für R³ genannten Bedeutungen besitzen, oder benachbarte Reste R⁴ bis R¹² mit den sie verbindenden Atomen einen oder mehrere aromatische oder aliphatische Ringe bilden, oder die Reste R⁵ und R⁸ oder R¹² mit den sie verbindenden Atomen einen aromatischen oder aliphatischen Ring bilden,
R¹³ 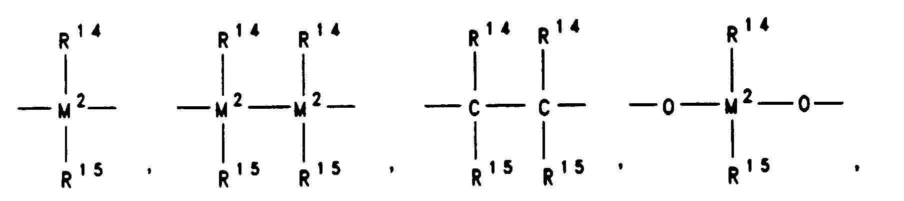 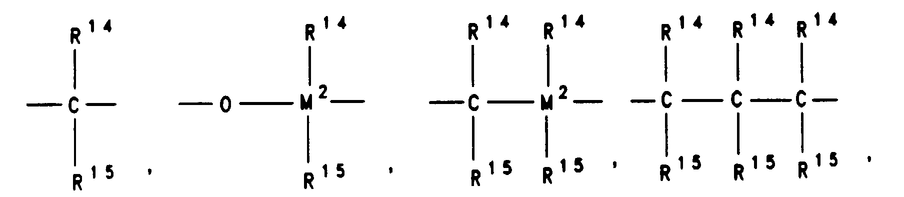 =BR¹⁴, =AIR¹⁴, -Ge-, -O-, -S-, =SO, =SO₂, =NR¹⁴, =CO, =PR¹⁴ oder =P(O)- R¹⁴ ist, wobei R¹⁴ und R¹⁵ gleich oder verschieden sind und ein Wasserstoffatom, ein Halogenatom, eine C₁-C₁₀-Alkyl-, eine C₁-C₁₀-Fluoralkyl-, eine C₁-C₁₀-Alkoxy-, eine C₆-C₁₀-Aryl-, eine C₆-C₁₀-Fluoraryl-, eine C₆-C₁₀-Aryloxy-, eine C₂-C₁₀-Alkenyl-, eine C₇-C₄₀-Arylalkyl-, eine C₇-C₄₀-Alkylaryl-, eine C₈-C₄₀-Arylalkenylgruppe bedeuten, oder R¹⁴ und R¹⁵ jeweils mit den sie verbindenden Atomen einen oder mehrere Ringe bilden und
M² Silizium, Germanium oder Zinn ist.

2. Verbindung der Formel I gemäß Anspruch 1, dadurch gekennzeichnet, daß in der Formel I
M¹ Zirkonium oder Hafnium ist,
R¹ und R² gleich sind und eine C₁-C₃-Alkylgruppe oder ein Halogenatom bedeuten,
die Reste R³ gleich sind und eine C₁-C₄-Alkylgruppe darstellen,
R⁴ bis R¹² gleich oder verschieden sind und Wasserstoff oder eine C₁- C₄-Alkylgruppe bedeuten und
R¹³ für 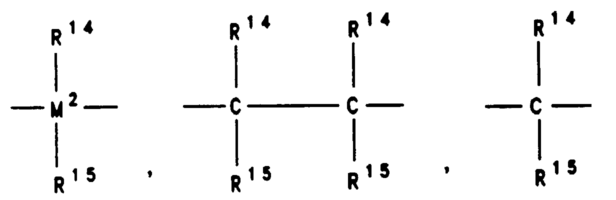 steht, wobei M² Silizium oder Germanium ist und R¹⁴ und R¹⁵ gleich oder verschieden sind und für eine C₁-C₄- Alkylgruppe oder eine C₆-C₁₀-Arylgruppe stehen.

3. Verbindung der Formel I gemäß Anspruch 1, dadurch gekennzeichnet, daß in der Formel I
R⁴ und R⁷ Wasserstoff bedeuten und
R⁵, R⁶ und R⁸ bis R¹² gleich oder verschieden sind und Wasserstoff oder eine C₁- C₄-Alkylgruppe bedeuten.

4. Verbindung der Formel I gemäß Anspruch 1, dadurch gekennzeichnet, daß in der Formel I
M¹ Zirkonium ist,
R¹ und R² gleich sind und Chlor bedeuten,
die Reste R³ gleich sind und eine C₁-C₄-Alkylgruppe bedeuten,
R⁴ und R⁷ Wasserstoff sind,
R⁵, R⁶ und R⁸ bis R¹² gleich oder verschieden sind und eine C₁-C₄- Alkylgruppe oder Wasserstoff bedeuten und
R¹³ für 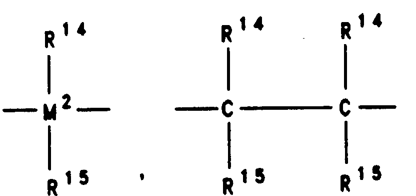 steht, wobei M² Silizium ist und R¹⁴ und R¹⁵ gleich oder verschieden sind und für eine C₁-C₄-Alkylgruppe oder eine C₆-C₁₀-Arylgruppe stehen.

5. Verbindung der Formel I gemäß einem oder mehreren der Ansprüche 1 bis 4, dadurch gekennzeichnet, daß in Formel I
M¹ Zirkonium, R¹ und R² Chlor sind, die Reste R³ Methyl oder Ethyl bedeuten,
R⁴ bis R¹² Wasserstoff bedeuten und
R¹³ für 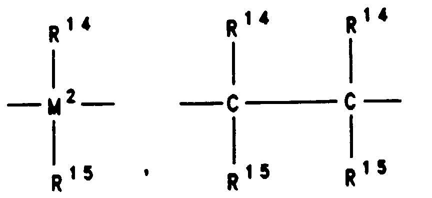 steht, wobei M² Silizium bedeutet, und
R¹⁴ und R¹⁵ gleich oder verschieden sind und Methyl, Ethyl, n-Propyl, i-Propyl oder Phenyl bedeuten.

6. Verfahren zur Herstellung eines Olefinpolymers durch Polymerisation oder Copolymerisation eines Olefins der Formel R^{a}-CH=CH-R^{b}, worin R^{a} und R^{b} gleich oder verschieden sind und ein Wasserstoffatom oder einen Kohlenwasserstoffrest mit 1 bis 14 C-Atomen bedeuten, oder R^{a} und R^{b} mit den sie verbindenden Atomen einen oder mehrere Ringe bilden können, bei einer Temperatur von -60 bis 200°C, bei einem Druck von 0,5 bis 100 bar, in Lösung, in Suspension oder in der Gasphase, in Gegenwart eines Katalysators, welcher aus einem Metallocen als Übergangsmetallverbindung und einem Cokatalysator gebildet wird, dadurch gekennzeichnet, daß das Metallocen eine Verbindung der Formel I gemäß Anspruch 1 ist.

7. Verfahren gemäß Anspruch 6, dadurch gekennzeichnet, daß als Cokatalysator ein Aluminoxan der Formel IIa für den linearen Typ und/oder der Formel IIb für den cyclischen Typ 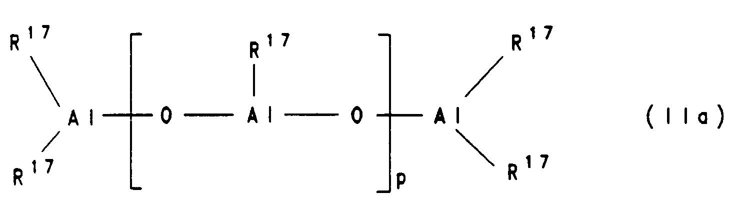 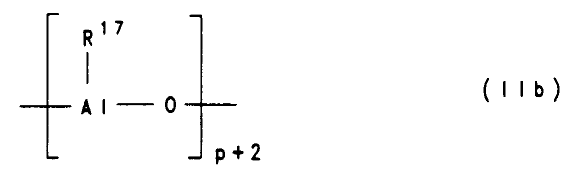 verwendet wird, wobei in den Formeln lla und llb die Reste R¹⁷ gleich oder verschieden sind und eine C₁-C₆- Alkylgruppe, eine C₆-C₁₈-Arylgruppe, Benzyl oder Wasserstoff bedeuten und p eine ganze Zahl von 2 bis 50 ist.

8. Verfahren gemäß Anspruch 6, dadurch gekennzeichnet, daß als Cokatalysator Methylaluminoxan verwendet wird.

9. Verfahren gemäß Anspruch 6, dadurch gekennzeichnet, daß das Metallocen der Formel I vor dem Einsatz in der Polymerisationsreaktion mit einem Aluminoxan der Formel lla und/oder llb voraktiviert wird.

10. Verfahren gemäß Anspruch 6, dadurch gekennzeichnet, daß ein geträgerter Polymerisations-Katalysator eingesetzt wird, der das Reaktionsprodukt eines Metallocens der Formel I mit einer geträgerten Organoaluminium-Verbindung (Cokatalysator) ist.

11. Verfahren gemäß Anspruch 10, dadurch gekennzeichnet, daß das Trägermaterial ein Oxid des Siliziums und/oder Aluminiums ist und die Organoaluminium-Verbindung Methylaluminoxan ist.

12. Verwendung eines Metallocens der Formel I gemäß Anspruch 1 als Katalysatorkomponente bei der Polymerisation oder Copolymerisation von Olefinen.

## Claims

1. A compound of the formula I 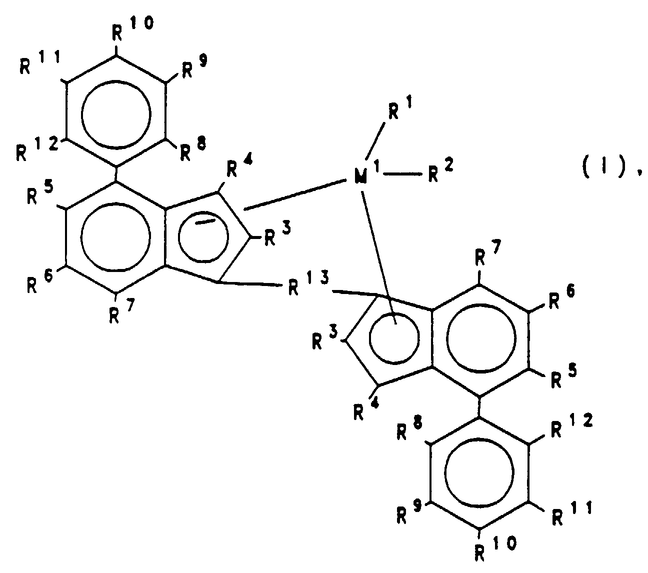 in which
M¹ is a metal from group IVb, Vb or VIb of the Periodic Table,
R¹ and R² are identical or different and are a hydrogen atom, a C₁-C₁₀-alkyl group, a C₁-C₁₀-alkoxy group, a C₆-C₁₀-aryl group, a C₆-C₁₀-aryloxy group, a C₂-C₁₀-alkenyl group, a C₇-C₄₀-arylalkyl group, a C₇-C₄₀-alkylaryl group, a C₈-C₄₀-arylalkenyl group, an OH group or a halogen atom,
the radicals R³ are identical or different and are a hydrogen atom, a halogen atom, a C₁-C₁₀-alkyl group, which may be halogenated, a C₆-C₁₀-aryl group, an -NR¹⁶₂, -SR¹⁶, -OSiR¹⁶₃, -SiR¹⁶₃ or -PR¹⁶₂ radical, in which R¹⁶ is a halogen atom, a C₁-C₁₀-alkyl group or a C₆-C₁₀-aryl group,
R⁴ to R¹² are identical or different and are as defined for R³, or adjacent radicals R⁴ to R¹², together with the atoms connecting them, form one or more aromatic or aliphatic rings, or the radicals R⁵ and R⁸ or R¹², together with the atoms connecting them, form an aromatic or aliphatic ring,
R¹³ is 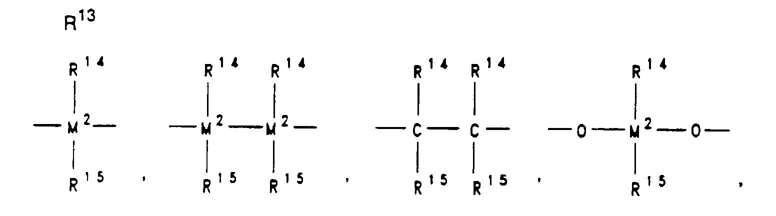 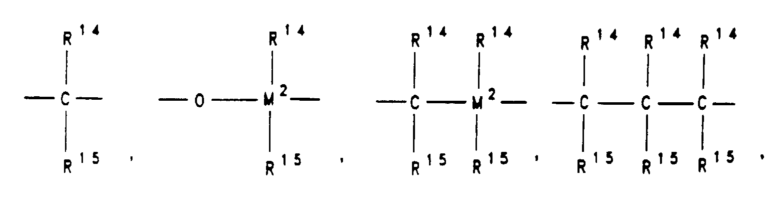 =BR¹⁴, =AlR¹⁴, -Ge-, -O-, -S-, =SO, =SO₂, =NR¹⁴, =CO, =PR¹⁴ or =P(O)R¹⁴, where R¹⁴ and R¹⁵ are identical or different and are a hydrogen atom, a halogen atom, a C₁-C₁₀-alkyl group, a C₁-C₁₀-fluoroalkyl group, a C₁-C₁₀-alkoxy group, a C₆-C₁₀-aryl group, a C₆-C₁₀-fluoroaryl group, a C₆-C₁₀-aryloxy group, a C₂-C₁₀-alkenyl group, a C₇-C₄₀-arylalkyl group, a C₇-C₄₀-alkylaryl group or a C₈-C₄₀-arylalkenyl group, or R¹⁴ and R¹⁵, in each case together with the atoms connecting them, form one or more rings, and
M² is silicon, germanium or tin.

2. A compound of the formula I as claimed in claim 1, wherein, in the formula I,
M¹ is zirconium or hafnium,
R¹ and R² are identical and are a C₁-C₃-alkyl group or a halogen atom,
the radicals R³ are identical and are a C₁-C₄-alkyl group,
R⁴ to R¹² are identical or different and are hydrogen or a C₁-C₄-alkyl group, and
R¹³ is 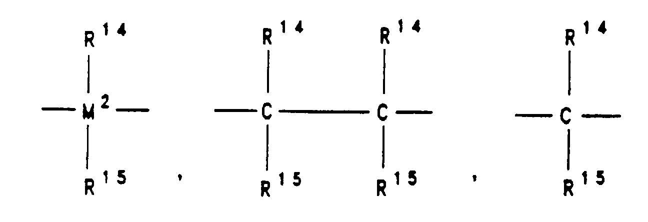 where M² is silicon or germanium and R¹⁴ and R¹⁵ are identical or different and are a C₁-C₄-alkyl group or a C₆-C₁₀-aryl group.

3. A compound of the formula I as claimed in claim 1, wherein, in the formula I,
R⁴ and R⁷ are hydrogen, and
R⁵, R⁶ and R⁸ to R¹² are identical or different and are hydrogen or a C₁-C₄-alkyl group.

4. A compound of the formula I as claimed in claim 1, wherein, in the formula I,
M¹ is zirconium,
R¹ and R² are identical and are chlorine,
the radicals R³ are identical and are a C₁-C₄-alkyl group,
R⁴ and R⁷ are hydrogen,
R⁵, R⁶ and R⁸ to R¹² are identical or different and are a C₁-C₄-alkyl group or hydrogen, and
R¹³ is 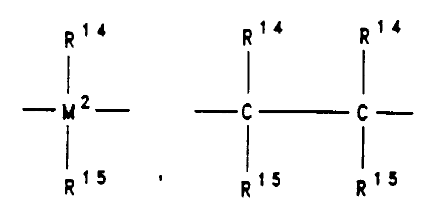 where M² is silicon and R¹⁴ and R¹⁵ are identical or different and are a C₁-C₄-alkyl group or a C₆-C₁₀-aryl group.

5. A compound of the formula I as claimed in one or more of claims 1 to 4, wherein, in the formula I, M¹ is zirconium, R¹ and R² are chlorine, the radicals R³ are methyl or ethyl, R⁴ to R¹² are hydrogen, and
R¹³ is 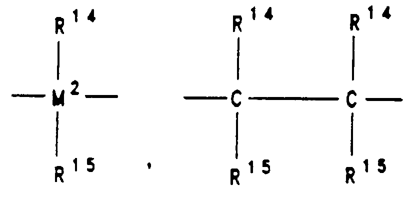 where M² is silicon, and
R¹⁴ and R¹⁵ are identical or different and are methyl, ethyl, n-propyl, i-propyl or phenyl.

6. A process for the preparation of an olefin polymer by polymerization or copolymerization of an olefin of the formula R^{a}-CH=CH-R^{b}, in which R^{a} and R^{b} are identical or different and are a hydrogen atom or a hydrocarbon radical having 1 to 14 carbon atoms, or R^{a} and R^{b}, together with the atoms connecting them, can form one or more rings, at a temperature of from -60 to 200°C, at a pressure of 0.5 to 100 bar, in solution, in suspension or in the gas phase, in the presence of a catalyst formed from a metallocene as transition-metal compound and a cocatalyst, wherein the metallocene is a compound of the formula I as claimed in claim 1.

7. The process as claimed in claim 6, wherein the cocatalyst used is an aluminoxane of the formula IIa for the linear type and/or of the formula IIb for the cyclic type 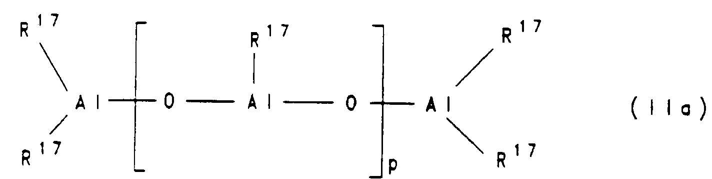 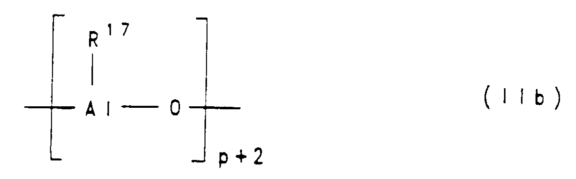 where, in the formulae IIa and IIb, the radicals R¹⁷ are identical or different and are a C₁-C₆-alkyl group, a C₆-C₁₈-aryl group, benzyl or hydrogen, and p is an integer from 2 to 50.

8. The process as claimed in claim 6, wherein the cocatalyst used is methylaluminoxane.

9. The process as claimed in claim 6, wherein the metallocene of the formula I is preactivated by means of an aluminoxane of the formula IIa and/or IIb before use in the polymerization reaction.

10. The process as claimed in claim 6, wherein a supported polymerization catalyst is employed which is the product of the reaction of a metallocene of the formula I with a supported organoaluminum compound (cocatalyst).

11. The process as claimed in claim 10, wherein the support material is an oxide of silicon and/or of aluminum, and the organoaluminum compound is methylaluminoxane.

12. The use of a metallocene of the formula I as claimed in claim 1 as a catalyst component in the polymerization or copolymerization of olefins.

## Revendications

1. Composé de formule I : 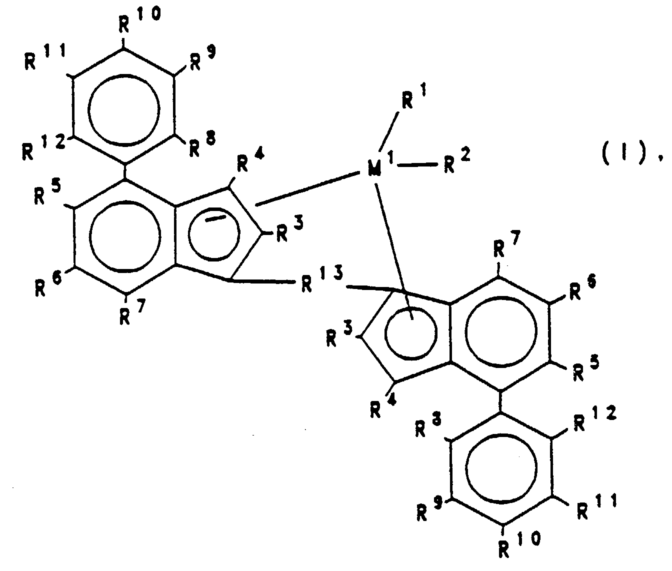 dans laquelle :
laquelle :
M¹ est un métal du groupe IVb, Vb ou VIb du Tableau Périodique,
R¹ et R² sont identiques ou différents et représentent chacun un atome d'hydrogène, un groupe alkyle en C₁-C₁₀, un groupe alcoxy en C₁-C₁₀, aryle en C₆-C₁₀, aryloxy en C₆-C₁₀, alcényle en C₂-C₁₀, arylalkyle en C₇-C₄₀, alkylaryle en C₇-C₄₀, arylalcényle en C₈-C₄₀, un groupe OH ou un atome d'halogène,
les radicaux R³ sont identiques ou différents et représentent chacun un atome d'hydrogène, un atome d'halogène, un groupe alkyle en C₁-C₁₀, pouvant être halogéné, un groupe aryle en C₆-C₁₀, un radical -NR¹⁶₂, -SR¹⁶, -OSiR¹⁶₃, SiR¹⁶₃ ou -PR¹⁶₂, où R¹⁶ est un atome d'halogène, un groupe alkyle en C₁-C₁₀, ou un groupe aryle en C₆-C₁₀,
R⁴ à R¹² sont identiques ou différents et ont les significations données pour R³, ou encore des radicaux R⁴ à R¹² voisins forment avec les atomes qui les relient un ou plusieurs noyaux aromatiques ou aliphatiques, ou encore les radicaux R⁵ et R⁸ ou R¹² forment avec les atomes qui les relient un noyau aromatique ou aliphatique,
R¹³ est : 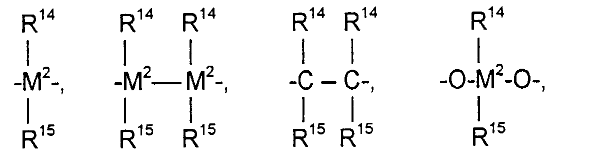 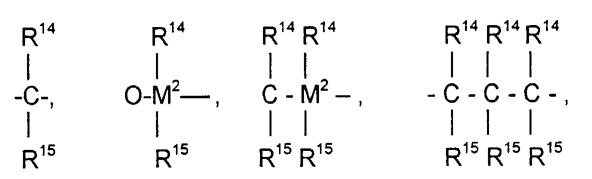 =BR¹⁴, =AlR¹⁴, -Ge-, -O-, -S-, =SO, =SO₂, =NR¹⁴, =CO, =PR¹⁴ ou =P(O)R¹⁴,
où :
R¹⁴ et R¹⁵ sont identiques ou différents et représentent chacun un atome d'hydrogène, un atome d'halogène, un groupe alkyle en C₁-C₁₀, fluoralkyle en C₁-C₁₀, alcoxy en C₁-C₁₀, aryle en C₆-C₁₀, fluoraryle en C₆-C₁₀, aryloxy en C₆-C₁₀,alcényle en C₂-C₁₀, arylalkyle en C₇-C₄₀, alkylaryle en C₇-C₄₀, arylalcényle en C₈-C₄₀, ou encore R¹⁴ et R¹⁵, avec les atomes qui les relient, forment un ou plusieurs noyaux, et
M² est le silicium, le germanium ou l'étain.

2. Composé de formule I selon la revendication 1, caractérisé en ce que, dans la formule I :
M¹ est le zirconium ou le hafnium,
R¹ et R² sont identiques et représentent chacun un groupe alkyle en C₁-C₃, ou un atome d'halogène,
les radicaux R³ sont identiques et représentent chacun un groupe alkyle en C₁-C₄,
R⁴ à R¹² sont identiques ou différents et représentent chacun un hydrogène ou un groupe alkyle en C₁-C₄,
R¹³ est : 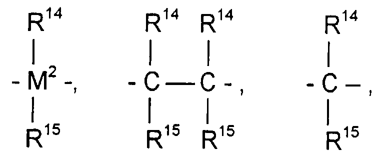 où :
M² est le silicium ou le germanium, et R¹⁴ et R¹⁵ sont identiques ou différents et représentent chacun un groupe alkyle en C₁-C₄, ou un groupe aryle en C₆-C₁₀.

3. Composé de formule I selon la revendication 1, caractérisé en ce que, dans la formule I,
R⁴ et R⁷ sont des atomes d'hydrogène, et
R⁵, R⁶ et R⁸ à R¹² sont identiques ou différents et représentent chacun un atome d'hydrogène ou un groupe alkyle en C₁-C₄.

4. Composé de formule I selon la revendication 1, caractérisé en ce que, dans la formule I :
M¹ est le zirconium,
R¹ et R² sont identiques et représentent chacun le chlore,
les radicaux R³ sont identiques et représentent chacun un groupe alkyle en C₁-C₄,
R⁴ et R⁷ sont des atomes d'hydrogène,
R⁵, R⁶ et R⁸ à R¹² sont identiques ou différents et représentent chacun un groupe alkyle en C₁-C₄ ou un atome d'hydrogène, et
R¹³ est : 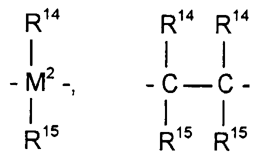 où :
M² est le silicium, et R¹⁴ et R¹⁵ sont identiques ou différents et représentent chacun un groupe alkyle en C₁-C₄, ou un groupe aryle en C₆-C₁₀.

5. Composé de formule I selon l'une ou plusieurs des revendications 1 à 4, caractérisé en ce que, dans la formule I :
M¹ est le zirconium,
R¹ et R² sont des atomes de chlore,
les radicaux R³ sont des groupes méthyle ou éthyle R⁴ et R¹² sont des atomes d'hydrogène, et
R¹³ est : 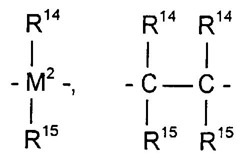
où :
M² est le silicium, et
R¹⁴ et R¹⁵ sont identiques ou différents et représentent chacun le groupe méthyle, éthyle, n-propyle, isopropyle ou phényle.

6. Procédé de préparation d'un polymère oléfinique par polymérisation ou copolymérisation d'une oléfine de formule R^{a}-CH=CH-R^{b}, ou R^{a} et R^{b} sont identiques ou différents et représentent chacun un atome d'hydrogène ou un radical hydrocarboné ayant de 1 à 14 atomes de carbone, ou bien R^{a} et R^{b}, avec les atomes qui les relient, peuvent former un ou plusieurs noyaux, à une température de -60 à 200°C, sous une pression de 0,5 à 100 bar, en solution, en suspension ou en phase gazeuse, en présence d'un catalyseur qui est formé à partir d'un métallocène servant de composé d'un métal de transition et d'un cocatalyseur, caractérisé en ce que le métallocène est un composé de formule I selon la revendication 1.

7. Procédé selon la revendication 6, caractérisé en ce qu'on utilise comme co-catalyseur un aluminoxane de formule IIa pour le type linéaire et/ou de formule IIb pour le type cyclique : 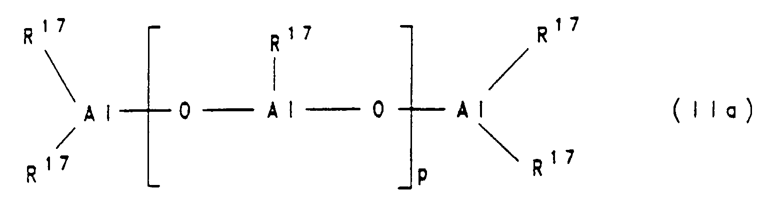 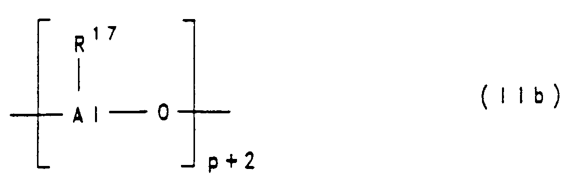 où :
dans les formules IIa et IIb, les radicaux R¹⁷ sont identiques ou différents et représentent chacun ou groupe alkyle en C₁-C₆, un groupe aryle en C₆-C₁₈, benzyle ou un hydrogène, et p est un nombre entier de 2 à 50.

8. Procédé selon la revendication 6, caractérisé en ce qu'on utilise du méthylaluminoxane en tant que co-catalyseur.

9. Procédé selon la revendication 6, caractérisé en ce que le métallocène de formule I est, avant utilisation dans la réaction de polymérisation, préactivé avec un aluminoxane de formule IIa/IIb.

10. Procédé selon la revendication 6, caractérisé en ce qu'on utilise un catalyseur supporté de polymérisation, qui est le produit de la réaction d'un métallocène de formule I avec un composé organoaluminié supporté (co-catalyseur).

11. Procédé selon la revendication 10, caractérisé en ce que le matériau support est un oxyde de silicium et/ou d'aluminium, et que le composé organoaluminié est le méthylaluminoxane.

12. Utilisation d'un métallocène de formule I selon la revendication 1 en tant que constituant catalyseur lors de la polymérisation ou de la copolymérisation d'oléfines.
